# EUROPEAN PATENT APPLICATION

(11) **EP 1 061 129 A1**
(43) Date of publication of application: **20.12.2000**
(21) Application number: 99401199.7
(22) Date of filing: 18.05.1999
(51) Int. Cl.: C12N 7/00, C12N 15/48, C12N 15/86, C12N 5/08, C07K 14/15, A61K 35/76, A61K 38/16, A61K 48/00, C12Q 1/68, G01N 33/569, A61P 35/00, A61P 37/00

(54) **Infectious retroviruses from a leukemic dog cell line with extensive homologies to murine leukemia viruses**

(71) Applicant: Etablissement de Transfusion Sanguine de Lyon, 69342 Lyon Cédex 07 (FR)
(72) Inventor: The designation of the inventor has not yet been filed
(74) Representative: Dufresne, Guillaume Alain François

(57) **Abstract**

Retrovirus characterized in that :
- it is of type C morphology ;
- it sediments in a sucrose gradient at a density comprised between 1.16 g/l and 1.18 g/l ;
- it is infectious for dog cells ; and
- it belongs to the oncovirinae group.

## Description

Retrovirus have been isolated from a number of mammalian species. Surprisingly, until now no canine retrovirus has been characterized, despite the fact that RT activity has been found in supernatants of dog lymphosarcoma cell lines (23) and (36).

The difficulties met until now, have been to establish or maintain in culture a dog cell line able to produce retroviral particles. Said problem is for the first time and despite many people have unsuccessfully tried solved by the present invention which provides :

A lymphoma T-CD8⁺ cell line called DLC 01 which is derived from a fresh lymph node of a dog suffering from a cutaneous CD8⁺ T lymphoma (Sézary syndrome). DLC 01 cell lines have been registered at the CNCM the 17^{th} May 1999 under the registry number I-2200.

When observed by electron microscopy, this cell line produce extracellular particles highly suggestive of a retrovirus. This infectious retrovirus isolated from a canine T CD8⁺ lymphoma cell has been characterized.

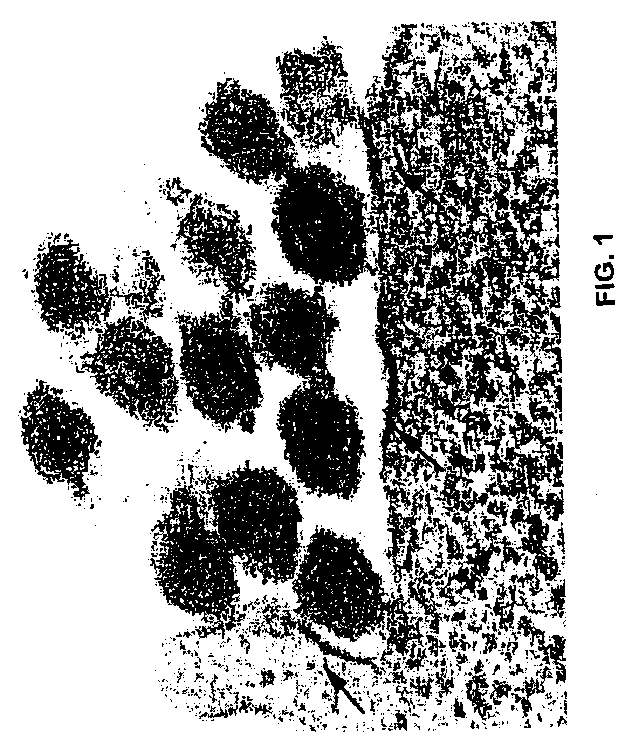

As shown in Figure 1, the diameter of these spherical particles is about 100 nm, and they appear to derive from thickening areas at the cell membrane. Furthermore, these extracellular viral particles show a type C morphology.

These viruses have been found to have a sucrose gradient density of around 1.17 g/l. These viruses have all the ultrastructural and biochemical characteristics of a mammalian type C oncovirus. Their reverse transcriptase activity presents an Mn²⁺ preference, and use tRNApro as the primer for initiation of minus strand cDNA synthesis. Said reverse transcriptase activity is typical of viruses belonging to the mammalian type C oncoviruses which are members of the family Retroviridae (7), (9). Virions produced by the DLC 01 cells are able to infect canine, human and murine cells in culture.

Sequence analysis of RNA isolated from virions after RT-PCR using oligonucleotides which anneal to sequences conserved among mammalian type C oncoviruses shows that these isolates are highly related to Murine Leukemia Viruses (MLVs), and particularly to Akv. MLVs are widespread within vertebrates as both endogenous elements and exogenous agents (22) and a number of them have been associated with numerous pathogenic effects, such as malignancies, mainly leukemia and lymphoma (15), (10) immunosuppression, neurological disorders (11) or inflammatory diseases. Many of these exogenous retroviruses possess nucleotide sequences similar to endogenous proviruses, which are carried within the host genome and are therefore, vertically transmitted. These endogenous type elements appear to be ubiquitous within mammals, including humans, since they have been been isolated from a large number of different taxa (22), (18). The murine leukemia viruses have been classified by competitive viral interference patterns which are defined by their receptor specificities (4), (26), (30). Viral envelope glyco-proteins can bind to a cognate host cell receptor and block superinfection of a virus which utilizes the same receptor. The main groups defined are ecotropic, amphotropic, xenotropic and polytropic.

The invention concerns a retrovirus characterized in that :
- it is of type C morphology ; and/or
- it sediments in a sucrose gradient at a density comprised between 1.1 to 1.2 g/l ; and/or
- it is infectious for dog cells ; and/or
- it belongs to the oncovirinae group.

The invention concerns a retrovirus characterized in that :
- it is of type C morphology ;
- it sediments in a sucrose gradient at a density comprised between 1.1 to 1.2 g/l ;
- it is infectious for dog cells ; and
- it belongs to the oncovirinae group.

The term « retrovirus » may encompass para-retrovirus and retro-transposon.

A virus having a « type C morphology » may be a virus which presents spikes at its surface, a central dense core and which appears to derive from thickenings of the cell membrane.

The « sedimentation density of a virus in a sucrose gradient » may be measured or carried out as described in example 2.

The « oncovirinae group » of viruses may be defined as the group of viruses which may be found in several animal groups, which are frequently associated with various forms of cancer.

The term « infectious » may mean in the context of the invention an ability :
- to replicate in the target cell, and/or
- to adsorb at the surface of the target cell, and/or
- to interact with its sub-unit SU of its envelope protein with a receptor present at the membrane of the target cell, and/or
- to begin to fusion by its viral membrane with the membrane of the target cell, and/or
- to liberate its capsid into the cytoplasmic compartment of the target cell, and/or
- to enter the target cell by endocytosis, and/or
- to intiate a reverse transcription into the target cell, and/or
- to generate a proviral DNA which is able to translocate into the nucleus of the target cell, and/or
- to generate a proviral DNA which is able to integrate in the DNA of the target cell, and/or
- to generate an integrated proviral which is able to be transcribed into RNA, and/or
- to generate a transcribed RNA inside the target cell, said RNA being able to be maturated in the cytoplasm of the target cell, and/or
- to generate a transcribed RNA inside the target cell, said RNA being translated into polyproteic precursors which are coded by the gag gene, and/or are coded by the gag-pol gene and/or are the precursor Pr65^{gag} and/or are the precursor Pr80^{env}, and/or are the precursor Pr180^{gagpol}, and/or
- to generate a transcribed RNA inside the target cell, said RNA being being able to produce the SU and TM (pl5E) sub-units, and/or
- to generate an in the target cell transcribed genomic RNA which is able to be encapsidated, and/or
- to generate after one or more viral replication cycle in the target cell viral particles which derive from thickenings of the target cell membrane or which have the ultrastructural and/or biochemical characteristics of a mammalian type C oncovirus or of a type C retrovirus, and/or
- to generate after one or more viral replication cycle in the target cell viral particles which are mature, and/or whose proteases are activated during the budding of the viral particle, and/or whose gag, gagpol and env are processed by the viral protease, and/or whose fusion activity of the TM is activated by proteolytic cleavage, and/or

In order to determine if a virus is « infectious » for a specific target cell, the following methods and measurement may be used :

A method consists in determining if the virus is able to produce an RT activity measurable in the supernatant of a target cell which has been put into contact with the virus. In said method, the putting into contact with the virus is preferably done in the absence of any agent facilitating or inducing « infectious » property of a virus, especially of a retrovirus and more especially of a mammalian type C oncovirus. An agent facilitating or inducing « infections » property may mean a transfecting agent.

Generally, methods and measurement to determine if a virus is « infectious » for a specific target cell, may preferably be done in the absence of any agent facilitating or inducing « infectious » property of a virus, especially of a retrovirus and more especially of a mammalian type C oncovirus.

In the methods cited above, the target cell may preferably be put into contact with the virus in a coculture. In this case, the target cell is cocultured before measuring RT activity in the surpernatant of the coculture. In a coculture experiment, the target cell is preferably cultured with cells producing the virus whose « infectious » properties are tested. The coculture may advantageously be done as described in example 5 below.

The « dog cells » recited in this application may preferably be chosen among dog lymphocytes, CD8⁺ tumoral dog cells, dog cells isolated from a dog suffering from a cutaneous lymphoma, DLC 01 cells (registered at the CNCM the 17^{th} May, 1999 under registry number I-2200) DH82 and/or D17 dog cells.

A retrovirus of the invention may belong to the mammalian type C oncovirus group. Said group of virus comprises for example the MLV, FeLV, GALV, BaEV, RD114 and REV-A viruses.

A retrovirus of the invention may sediment in a sucrose gradient at a density comprised between 1.16 and 1.18 g/l. Preferably, a retrovirus of the invention sediments in a sucrose gradient at a density of 1.17 g/l.

A retrovirus of the invention may be described as a spherical particle of about 100 nm in diameter.

A retrovirus of the invention may be derived or isolated from a dog suffering from a cutaneous CD8⁺ T lymphoma, from a lymph node of a dog with a Sezary syndrome, from a dog T-lymphocyte which grows continuously and whose supernatant presents a reverse tanscriptase activity, from a dog CD8⁺ and/or CD4⁺ and/or CD4⁻ CD8⁺ T-lymphocyte which grows continuously and whose supernatant presents a reverse tanscriptase activity.

In a preferred embodiment, a retrovirus of the invention is produced by the canine DLC 01 cell line deposited at the CNCM the 17^{th} May 1999. The canine DLC 01 cell line grows continuously, produces retroviral particles of type C, has been initially isolated from tumoral lymph node of a 13 years dog suffering from a Sézary syndrom and its supernatant presents a reverse tanscriptase activity.

The DLC 01 cell line is characterized in the FR 96 11231 patent.

A retrovirus of the invention may be infectious for mammalian cells. Mammalian cells may advantageously be murine or human cells.

The human cells may advantageously be the SupT1 T lymphoid cells, K562, CEM, MOLT-4, DAUDI and/or GIGOYE cells.

The murine cells may advantageously be the NIH/3T3 fibroblastic cells.

A retrovirus of the invention may encode an Mn²⁺ dependent reverse transcriptase or a reverse transcriptase which has an optimal polymerase activity in presence of Mn^{2+.}

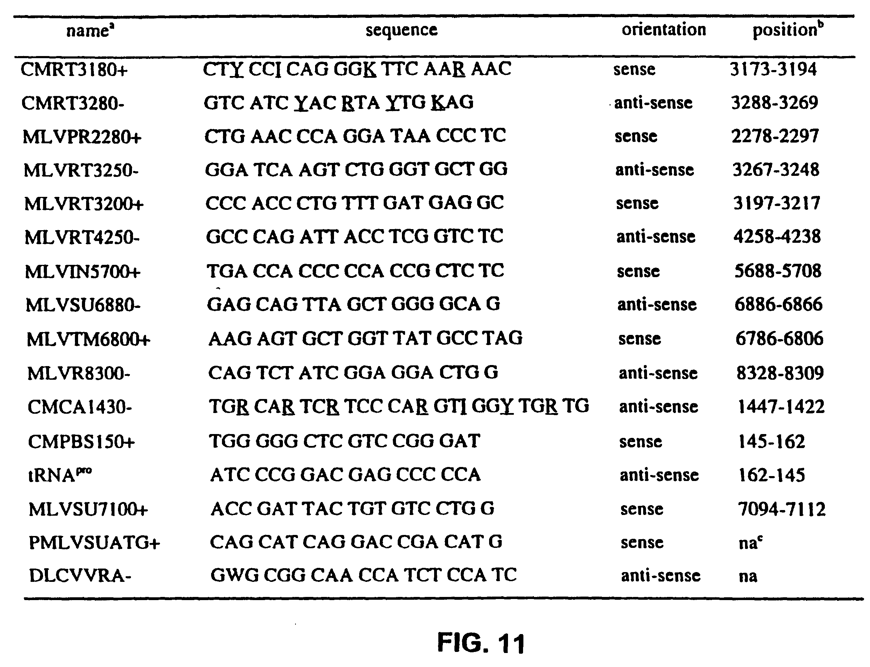

A retrovirus of the invention may use the tRNApro for the initiation of reverse transcription. tRNA^{pro} may be tRNA^{pro} depicted in figure 11. The initiation of reverse transcription by the tRNA^{pro} may be tested as described in example 4 below.

A retrovirus of the invention may produce a strong-stop DNA. Said strong-stop DNA may preferably be of 144 nucleotides long. The strong-stop DNA may be obtained by the technique described in example 4 below.

A retrovirus of the invention may be highly or closely homologous to Murine Leukemia Viruses (MLVs) in regards with its nucleotide sequence and/or amino acid sequence.

« Highly homologous» may mean a percentage of homology or identity comprised between about 70% and about 99%, or between about 80% and about 95%, or between about 90% and about 99%. A preferred percentage of homology or identity is 99%. The percentage of homology or identity may be determined with local align program.

In this application, a « fragment » or a « part » may mean a nucleic acid molecule of a length of at least 10, 20, 30, 40 or 50 nucleotides or a polypeptide of at least 3, 10, 20, 25 or 50 amino acids.

The « Murine Leukemia Viruses or MLV » cited in the application may be chosen among an E-tropic (Ecotropic), a P-tropic (Polytropic), a X-tropic (Xenotropic) or an A-tropic (Amphotropic) MLV virus.

An MLV X-tropic virus is infectious and replicate in cells from other species than the murine species. An MLV E-tropic virus is infectious and replicate in murine cells only.

An MLV A-tropic virus is infectious in murine cells and in cells from other species than the murine species.

An MLV P-tropic virus is infectious in murine cells and in cells from other species than the murine species.

An MLV X-tropic virus may be NZB 9-1, Bxv-1 or DBA2 ascitis endogenous leukemia virus.

An MLV E-tropic virus may be Akv or MoMLV and preferably Akv.

An MLV A-tropic may be 4070A.

An MLV P-tropic virus may be MCF 247 or MUSMLVPA.

A retrovirus of the invention may comprise in its genome a gag region which is highly homologous to the gag region of an MLV virus.

In this application, a « gag region » may mean part of the genome which encodes the gag precursor or any fragment of this precursor.

A retrovirus of the invention may comprise in its genome a region encoding the matrice protein (MA) and/or the p12 protein, and/or the capsid protein (CA), and/or the nucleocapsid protein (NC) or a part thereof which is highly homologous to the respective region encoding the matrice protein (MA) and/or the p12 protein, and/or the capsid protein (CA), and/or the nucleocapsid protein (NC) or a part thereof of an MLV virus.

The amino acid sequence of the CA protein or a part thereof of a retrovirus of the invention may have a 99% homology with the CA protein of an Akv virus, and/or 81% homology with the CA protein of a MoMLV virus, and/or 78% homology with the CA protein of a F-MLV/FB29 virus.

A retrovirus of the invention may comprise in its genome a pol region which is highly homologous to the pol region of an MLV virus.

In this application, a « pol region » may mean part of the genome which encodes the pol precursor or any fragment of this precursor, or which encodes at least one of the Protease (PR), Reverse Transcriptase (RT) and Integrase (IN) proteins or a part thereof.

A retrovirus of the invention may comprise in its genome a region encoding the protease (PR), and/or the reverse transcriptase (RT) and/or the integrase (IN) or a part thereof which is highly homologous to the respective region encoding the protease (PR), and/or the reverse transcriptase (RT) and/or the integrase (IN)or a part thereof of an MLV virus.

The amino acid sequence of the PR and/or RT region of a retrovirus of the invention may have a 99% homology with the PR and/or RT region of an Akv virus, and/or 89.5% homology with the PR and/or RT region of a MoMLV virus, and/or 90% homology with the PR and/or RT region of a F-MLV/FB29 virus.

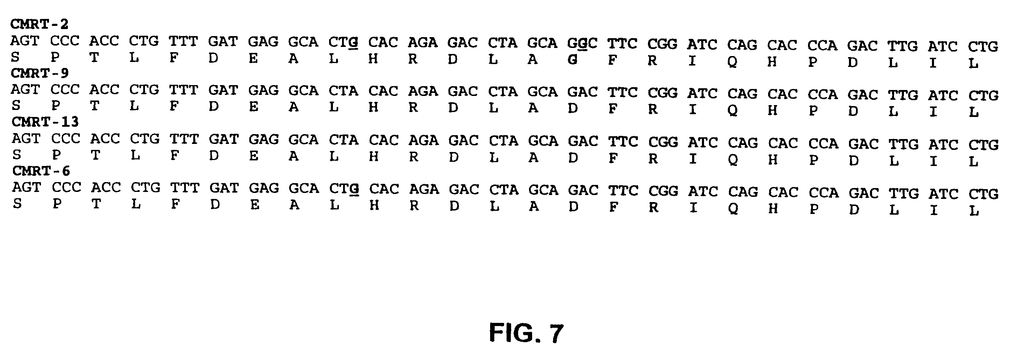

A retrovirus of the invention may preferably comprise in its genome a pol region or a part thereof whose cDNA comprises at least one of the DNA sequences depicted in figure 7.

A retrovirus of the invention may comprise in its genome an env region which is highly homologous to the env region of an MLV virus.

In this application, an « env region » may mean part of the genome which encodes the env precursor or any fragment of this precursor.

A retrovirus of the invention may comprise in its genome a region encoding the surface (SU) or transmembrane (TM) part of the enveloppe protein (env) or a part thereof which is highly homologous to the respective SU or TM region or a part thereof of an MLV virus.

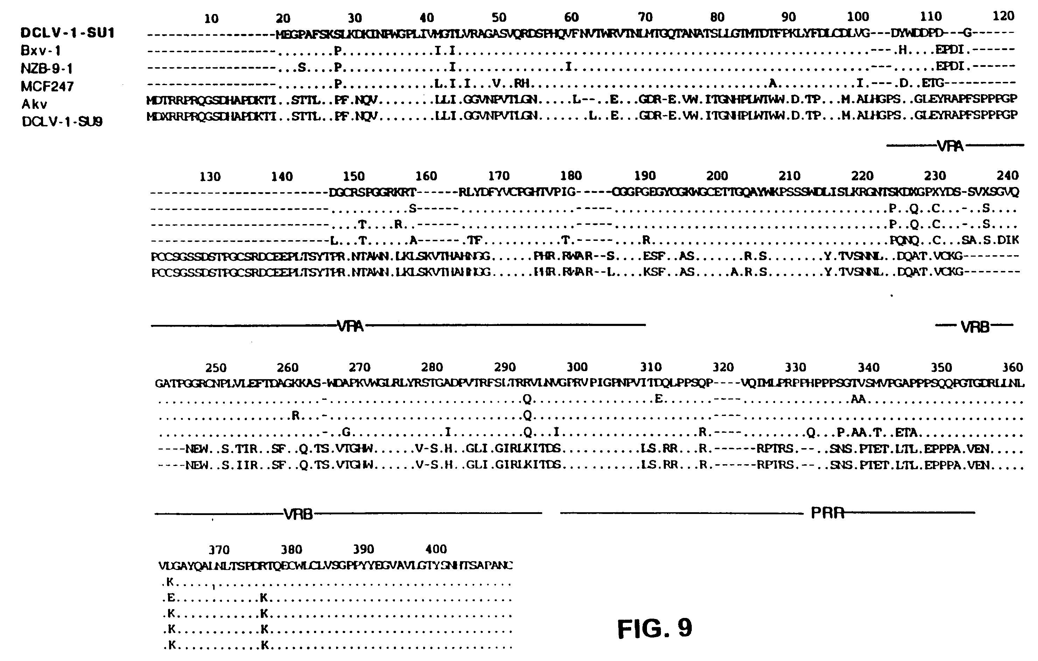

The SU region or surface part of the enveloppe protein (SU) is responsible for receptor binding (38), and the transmembrane part of the enveloppe protein (TM) anchors the envelope proteins at the membrane and induces membrane fusion during viral entry. Sequence comparisons of MLV envelopes show regions of strong conservation, such as the C-terminal domain of TM, while others are variable like the N-terminal domain. There are two hypervariable regions, called VRA and VRB, and a recognizable proline-rich region in the SU proteins (Fig. 9).

The amino acid sequence of the SU part of the env region of a retrovirus of the invention may have a 99% homology with the SU part of the env region of an Akv virus and/or 72% homology with the SU part of the env region of a MoMLV virus and/or 70% homology with the SU part of the env region of a F-MLV/FB29 virus.

A retrovirus of the invention may comprise in its genome a region which encodes at least part of the amino acid sequence designated as DCLV-1-SU9 depicted in figure 9, said part comprising at least one of the substitutions or deletions which distinguish DCLV-1-SU9 from Akv in figure 9 of this application.

A retrovirus of the invention defined in the preceding paragraph may advantageously encode all or part of the VRB region depicted in figure 9.

A retrovirus of the invention may preferably comprise in its genome a region which encodes the DCLV-1-SU9 sequence depicted in figure 9.

A retrovirus of the invention may comprise in its genome a region which encodes at least part of the amino acid sequence designated as DCLV-1-SU1 depicted in figure 9, said part comprising at least one of the substitutions or deletions which distinguish DCLV-1-SU1 from the Bxv-1, and/or from NZB-9-1 and/or from MCF247 sequences in figure 9 of this application.

A retrovirus of the invention defined in the preceding paragraph may advantageously encode all or part of the VRA region depicted in figure 9 and/or all or part of the VRB region depicted in figure 9.

A retrovirus of the invention may preferably comprise in its genome a region which encodes the DCLV-1-SU1 sequence depicted in figure 9.

A retrovirus of the invention may comprise in its genome a region encoding the SU part of the enveloppe protein or a fragment thereof whose cDNA comprises a PvuII fragment of about 1.6 kilobase pairs which contains a PvuII site.

A retrovirus of the invention may comprise in its genome a region encoding the SU part of the enveloppe protein or a fragment thereof which is a recombination between a P-tropic and a X-tropic MLV viruses. A MLV P-tropic virus may be MCF 247 or MUSMLVPA. A MLV X-tropic virus may be NZB 9-1, Bxv-1 or DBA2 ascitis endogenous leukemia virus.

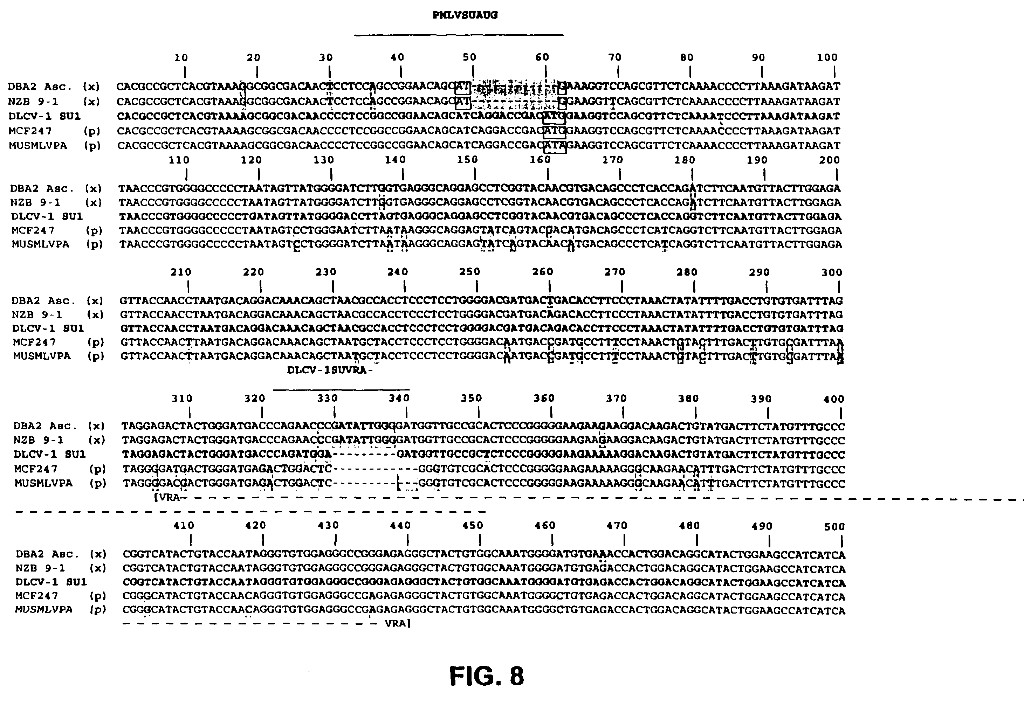

A retrovirus of the invention may comprise in its genome a region whose cDNA comprises at least a part of the amino acid sequence of the DCLV-1-SU1 sequence depicted in figure 8, said part comprising at least one of the substitution or deletion which distinguishes DCLV-1-SU1 from DBA2 asc and/or from NZB-9-1 and/or from MCF247 and/or from MUSMLPVA sequences depicted in figure 8 of this application.

A retrovirus of the invention defined in the preceding paragraph may advantageously comprise in the sequence of its cDNA all or part of the VRA region depicted in figure 8.

A retrovirus of the invention may preferably comprise in its genome a region whose cDNA sequence comprises the DCLV-1-SU1 sequence depicted in figure 8.

A retrovirus of the invention may present a 9-base pair deletion in the region encoding the variable region A (VRA) of the SU part of the env protein, when compared to an X-tropic MLV virus (see sequence alignment in Figure 8). A X-tropic MLV virus may preferably be DBA2 Asc and/or NZB 9-1 whose a part of the SU sequence is depicted in figure 8.

A retrovirus of the invention may present a 9-base pair deletion in the region encoding the variable region A (VRA) of the SU part of the env protein, when compared to the 12-base pair deletion of a P-tropic MLV virus (see sequence alignment in Figure 8). A P-tropic MLV virus may preferably be MCF247 and/or MUSMLVPA whose part of the SU sequence is depicted in figure 8.

A retrovirus of the invention may comprise a specific deletion in its genome in the region encoding the SU part of the env protein, or a fragment thereof, said deletion being situated at the major site of divergence between P-tropic and X-tropic MLV viruses.

A retrovirus of the invention may present 4 substitutions in its genome in the region encoding the SU part of the enveloppe protein, or a fragment thereof, when compared to the Akv virus.

A retrovirus of the invention may be an endogenous retrovirus. A retrovirus of the invention may comprise endogenous retroviral sequences present in the dog genome.

A retrovirus of the invention may immortalize dog cells. A retrovirus of the invention may be able to produce continuous dog cell lines. A retrovirus of the invention may be capable to produce dog cells capable of being maintained in culture more than one week, two weeks, three weeks, four weeks, two month, several weeks or several month. The dog cells may preferably be chosen among dog lymphocytes, CD8⁺ tumoral dog cells, dog cells isolated from a dog suffering from a cutaneous lymphoma, DLC 01 cells (deposited at the CNCM the 17^{th} May, 1999) and/or D17 dog cells.

A retrovirus of the invention may be any retrovirus which may be isolated and characterized from the DLC 01 cells registered at the CNCM the 17^{th} may 1999 under registry number I-2200.

In another aspect, the invention concerns any sub-unit which can be derived or isolated from any one of a retrovirus of the invention described above and any sub-unit which can be cloned and/or sequenced from any one of a retrovirus of the invention described above.

A sub-unit of the invention recited above may be a nucleic acid molecule or a polypeptide.

In a first embodiment, a sub-unit of the invention is a nucleic acid molecule.

A nucleic acid molecule of the invention may be isolated, cloned and/or sequenced from a retrovirus of the invention with known techniques. Said nucleic acid molecule of the invention may preferably be isolated, cloned and/or sequenced from a retrovirus of the invention with the techniques described in example 6 below.

A nucleic acid molecule of the invention may be an RNA molecule, a DNA molecule, a cDNA molecule, a single or double strand nucleic acid molecule, a proviral nucleic acid molecule, or a proviral DNA molecule or a combination thereof.

A nucleic acid molecule of the invention may have a length of at least 10 or 20 nucleotides.

A nucleic acid molecule of the invention may comprise in its sequence a sequence encoding a gag polyprotein or a part thereof which is highly homologous to the gag polyprotein of an MLV virus or to a part thereof. A nucleic acid molecule of the invention may comprise a MA, p12, CA or NC region or a part thereof which is highly homologous to the respective MA, p12, CA or NC region or a part thereof of an MLV virus.

« Highly homologous » as recited above and in the text below may mean homologous with a percentage of homology or identity comprised between about 70% and about 99%, or between about 80% and about 95%, or between about 90% and about 99%. A preferred percentage of homology or identity is 99%. The percentage of homology may be determined with local align program.

A nucleic acid molecule of the invention may comprise a sequence encoding the amino acid sequence of a CA protein or a part thereof which presents a 99% identity with the CA protein of an Akv virus or with a part thereof, 81% identity with the CA protein of a MoMLV virus or with a part thereof and/or 78% identity with the CA protein of a F-MLV/FB29 virus or with a part thereof.

A nucleic acid molecule of the invention may comprise in its sequence a sequence encoding an amino acid sequence of a pol polyprotein or a part thereof which is highly homologous to the pol polyprotein of an MLV virus or to a part thereof. A nucleic acid molecule of the invention may comprise a PR region and/or a RT region and/or a IN region or a part thereof which is highly homologous to the respective PR region and/or a RT region and/or a IN region or a part thereof of an MLV virus.

A nucleic acid molecule of the invention may comprise in its sequence a sequence encoding an amino acid sequence of a PR and/or RT region or of a part thereof which has 99% identity with the PR and/or RT region of an Akv virus or with a part thereof, which has 89.5% identity with the PR and/or RT region of a MoMLV virus or with a part thereof and/or which has 90% identity with the PR and/or RT region of a F-MLV/FB29 virus or with a part thereof.

A nucleic acid molecule of the invention may preferably comprise a pol region or a part thereof which comprises at least one of the DNA sequences depicted in figure 7 or a part thereof, said part comprising at least the codon which codes for the amino acid which differentiate the amino acid sequence of the CMRT-2 of figure 7 from the other amino acid sequences depicted in figure 7.

If a nucleic acid molecule of the invention recited in the preceding paragraph is a RNA molecule, the DNA sequences depicted in figure 7 or a part thereof recited in the preceding paragraph is replaced by a RNA sequence having the same nucleotidic sequence excepted that T nucleotide are replaced by U nucleotide.

A nucleic acid molecule of the invention may comprise in its sequence a sequence encoding an env protein or a part thereof which is highly homologous to the env protein of an MLV virus. A nucleic acid molecule of the invention may comprise a SU and/or TM region or a part thereof which is highly homologous to the respective SU or TM region or a part thereof of an MLV virus.

A nucleic acid molecule of the invention may comprise in its sequence a sequence encoding a SU region or a fragment thereof which has 99% homology with the SU region of an Akv virus or with a fragment thereof, which has 72% homology with the SU region of a MoMLV virus or with a fragment thereof and/or which has 70% homology with the SU region of a F-MLV/FB29 virus or with a fragment thereof.

A nucleic acid molecule of the invention may comprise in its sequence a sequence encoding a SU region or a fragment thereof which comprises at least part of the amino acid sequence of the sequence of the DCLV-1-SU9 depicted in figure 9, said part comprising at least one of the substitution or deletion which distinguishes DCLV-1-SU9 from Akv depicted in figure 9.

A « SU region » may mean a region of an env protein which is at the surface of the cell or outside the cell when the env protein is anchored in a cell membrane.

A nucleic acid molecule of the invention as defined in the preceding paragraph may advantageously encode all or part of the VRB region depicted in figure 9.

A nucleic acid molecule of the invention may preferably comprise a SU region, or a fragment thereof which encodes the DCLV-1-SU9 polypeptide depicted in figure 9.

A nucleic acid molecule of the invention may comprise in its sequence a sequence encoding a SU region, or a fragment thereof which comprises at least part of the amino acid sequence of the DCLV-1-SU1 depicted in figure 9, said part comprising at least one of the substitution or deletion which distinguishes DCLV-1-SU1 from the Bxv-1, and/or from NZB-9-1 and/or from MCF247 sequences depicted in figure 9.

A nucleic acid molecule of the invention as defined in the preceding paragraph may advantageously encode all or part of the VRA region depicted in figure 9 and/or all or part of the VRB region depicted in figure 9.

A nucleic acid molecule of the invention may preferably comprise a SU region or a fragment thereof which encodes the DCLV-1-SU1 polypeptide depicted in figure 9.

A nucleic acid molecule of the invention may preferably comprise a SU region or a fragment thereof which comprises a PvuII fragment of about 1.6 kilo base pairs which contains a PvuII site.

If a nucleic acid molecule of the invention recited in the preceding paragraph is a RNA molecule, the PvuII fragment of about 1.6 kilo base pairs recited in the preceding paragraph is replaced by a RNA sequence having the same nucleotidic sequence excepted that T nucleotides are replaced by U nucleotides.

A nucleic acid molecule of the invention may comprise a SU region or a fragment thereof which is a recombination between a SU region of a P-tropic MLV virus or a part thereof and a SU region of a X-tropic MLV virus or a fragment thereof. A MLV P-tropic virus may be MCF 247 or MUSMLVPA. A MLV X-tropic virus may be NZB 9-1, Bxv-1 or DBA2 ascitis endogenous leukemia virus.

A nucleic acid molecule of the invention may comprise a SU region or a fragment thereof which comprises at least a part of the nucleotide sequence of the DCLV-1-SU1 depicted in figure 8, said part comprising at least one of the substitution or deletion which distinguishes DCLV-1-SU1 from DBA2 asc and/or from NZB-9-1 and/or from MCF247 and/or from MUSMLPVA sequences depicted in figure 8.

A nucleic acid molecule of the invention defined in the preceding paragraph may advantageously comprise in its sequence all or part of the VRA region of the DLCV-1-SU1 sequence depicted in figure 8 and/or all or part of the VRB region of the DLCV-1-SU1 sequence depicted in figure 8.

A nucleic acid molecule of the invention may preferably comprise a SU region or a fragment thereof which comprises the DCLV-1-SU1 sequence depicted in figure 8.

If a nucleic acid molecule of the invention as recited in the three preceding paragraphs is a RNA molecule, the DCLV-1-SU1 depicted in figure 8, all or part of the VRA region of the DLCV-1-SU1 sequence depicted in figure 8 and all or part of the VRB region of the DLCV-1-SU1 sequence depicted in figure 8 are replaced by a RNA sequence having the same nucleotide sequence excepted that T nucleotides are replaced by U nucleotides.

A nucleic acid molecule of the invention may comprise a SU region or a fragment thereof which presents a 9-base pair deletion in the variable region A, when compared to an X-tropic MLV virus (see Figure 8). A X-tropic MLV virus may preferably be DBA2 Asc and NZB 9-1 depicted in figure 8.

A nucleic acid molecule of the invention may comprise a SU region or a fragment thereof which presents a 9-base pair deletion in the variable region A, when compared to the 12-base pair deletion of a P-tropic MLV virus (see Figure 8). A P-tropic MLV virus may preferably be MCF247 and/or MUSMLVPA depicted in figure 8.

A nucleic acid molecule of the invention may comprise a SU region or a fragment thereof which presents a specific deletion situated at the major site of divergence between the SU region of P-tropic and X-tropic MLV viruses (see Figure 8).

A nucleic acid molecule of the invention may comprise a SU region or a fragment thereof which presents 4 substitutions, when compared to the SU region of Akv virus.

A nucleic acid molecule of the invention may comprise in its sequence all or part of the sequence which can be retrieved from the cDNA of the RNA of a retrovirus of the invention by using a pair of primer chosen among the pairs of primers depicted in figure 11.

The invention concerns any derivative of a nucleic acid molecule of the invention described above or a part thereof.

A derivative of a nucleic acid molecule of the invention may be a nucleic acid molecule which comprises in its sequence the complementary sequence of the sequence of a nucleic acid molecule of the invention. A derivative of a nucleic acid molecule of the invention may be obtained by RT-PCR or by the in vitro action of a reverse transcriptase.

RT-PCR may be carried out as described in example 6.

In vitro action of a reverse transcriptase may be carried out as described in example 4.

A derivative of a nucleic acid molecule of the invention may be a nucleic acid molecule of the invention which has been modified in its composition or in its sequence in order to be able to have a specific use or function.

In a second embodiment, a sub-unit of the invention is a polypeptide. The term « polypeptide » may be replaced by the term « peptide » in the text below.

A polypeptide of the invention may be a polypeptide encoded by a nucleic acid molecule of the invention described above.

A polypeptide of the invention may be obtained or isolated from a retrovirus of the invention described above.

The isolation and/or identification of a polypeptide of the invention from a retrovirus of the invention described above may be carried out by gel migration or by immunoblot for example.

A polypeptide of the invention may comprise a pol region or a part thereof which comprises an amino acid sequence depicted in figure 7 or a part thereof, said part comprising at least the amino acid which differentiate the amino acid sequence of the CMRT-2 of figure 7 from the other amino acid sequences depicted in figure 7.

A polypeptide of the invention may comprise in its sequence a sequence encoding a SU region or a part thereof which has at least part of the amino acid sequence of the DCLV-1-SU9 sequence depicted in figure 9, said part comprising at least one of the substitution or deletion which distinguishes DCLV-1-SU9 sequence from Akv sequence depicted in figure 9.

A polypeptide of the invention as defined in the preceding paragraph may advantageously comprise all or part of the VRB region of DCLV-1-SU9 sequence depicted in figure 9.

A polypeptide of the invention may preferably comprise a SU region or a part thereof which comprises the DCLV-1-SU9 sequence depicted in figure 9.

A polypeptide of the invention may comprise in its sequence a sequence encoding a SU region or a fragment thereof which comprises at least a part of the amino acid sequence of the DCLV-1-SU1 sequence depicted in figure 9, said part comprising at least one of the substitution or deletion which distinguishes DCLV-1-SU1 from the Bxv-1, and/or from NZB-9-1 and/or from MCF247 sequences depicted in figure 9.

A polypeptide of the invention as defined in the preceding paragraph may advantageously comprise all or part of the VRA region of the DCLV-1-SU1 sequence depicted in figure 9 and/or all or part of the VRB region of the DCLV-1-SU1 sequence depicted in figure 9.

A polypeptide of the invention may preferably comprise a SU region or a part thereof which comprises the DCLV-1-SU1 sequence depicted in figure 9.

The invention concerns a vector comprising a nucleic acid molecule of the invention or a derivative thereof described above or a part thereof and a vector capable of expressing a polypeptide of the invention described above or a part thereof.

In a vector comprising a nucleic acid molecule of the invention or a derivative thereof, a nucleic acid molecule of the invention or a derivative thereof may be under the control of a regulatory sequence.

A vector of the invention may be a cloning vector, an expression vector or a pharmaceutical vector.

The invention concerns a cell or part of a cell which comprises a retrovirus of the invention, a nucleic acid molecule of the invention or a derivative thereof, or a vector of the invention.

The invention concerns a cell or part of a cell capable of expressing a polypeptide of the invention.

A cell of the invention may preferably be chosen among dog lymphocytes, CD8⁺ tumoral dog cells, dog cells isolated from a dog suffering from a cutaneous lymphoma, dog cells isolated from a dog suffering from a Sézary syndrom and DLC 01 cells (deposited at the CNCM the 17^{th} May, 1999 under registry number I-2200).

The invention concerns a use of a retrovirus of the invention, of a nucleic acid molecule of the invention or a derivative thereof, of a polypeptide of the invention or of a cell of the invention as a diagnostic tool.

The invention concerns a use of a retrovirus of the invention, of a nucleic acid molecule of the invention or of a derivative thereof ,of a polypeptide of the invention or of a cell of the invention as a diagnostic tool for a human or an animal.

The invention concerns a pharmaceutical composition comprising a retrovirus of the invention, a nucleic acid molecule of the invention or a derivative thereof or a polypeptide of the invention.

The invention concerns a use of a retrovirus of the invention, of a nucleic acid molecule of the invention or of a derivative thereof or of a polypeptide of the invention to implement a medicament to treat or prevent :
- an auto-immune disease ,
- an hematopoietic malignancy
- a malignant tumor,
- a disease associated with retrovirus and/or para-retrovirus infection,
- an infectious disease,
- a degenerative disease,
- a genetic disease,
- any one of the diseases listed above by somatic or germinal gene therapy.

Amplification of genomic RNA by RT-PCR reveals that RT coding sequences of the invention are closely related to Akv, an ecotropic virus known to only infect murine cells (34).

However PCR products of the invention amplified from the envelope region of a retrovirus of the invention are heterogeneous with the bulk of sequences, exemplified by the clones DLCV-1SU9, closely related to the E-tropic Akv envelope, and in DLCV-1 SU1, more related to X-tropic MLV envelopes. The presence of a mixture of envelopes interacting with different receptors may explain the remarkably broad host range of a retrovirus of the invention.

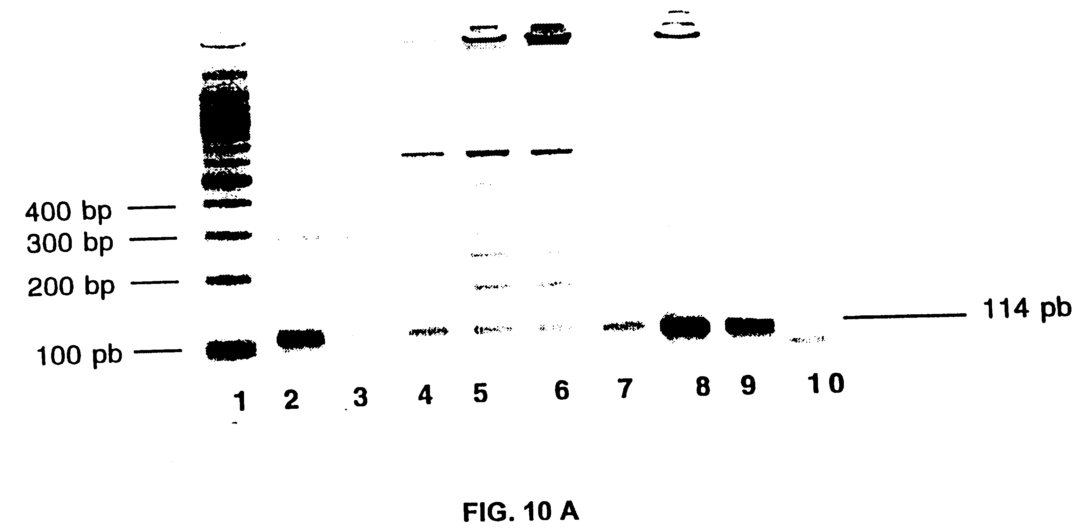

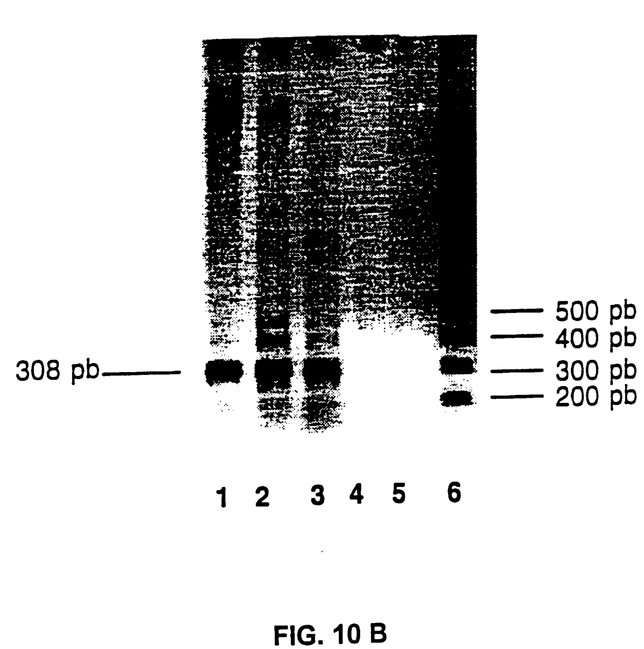

It is very surprising to observe that sequences between a retrovirus of the invention and MLV are so closely related. In the past, many isolates of a presumed new retrovirus in a new host later turned out to correspond to laboratory contaminations. Creation of the DLC 01 cell line, and the first ultrastructural studies showing type C retroviral particles, have been carried out in an immunology laboratory in which only human and canine biological materials are used, and are never virologically manipulated. The canine origin of DLC 01 cell line is verified by cytogenetic analysis, and we are able to amplify CAN-1 SINE sequences from the genomic DNA, which are specific for the canoidae family of mammals. Sequences identical to the Akv-related RT-PCR nucleic acid molecule of the invention obtained from DLC 01 supernatant can be amplified from primary cells derived from the dog from which this cell line is derived, which had never been passed in culture. As shown in Figure 10, the SU1 sequence of figure 8 could be amplified from genomic DNA of canine cells of various origins, but not from murine cells, indicating that this DLCV-01 SU1 envelope results from a recombination between canine endogenous retroviral sequences and an Akv-related retrovirus.

The presence of an Akv-related retrovirus in canine leukemic cells is surprising. It should be pointed out that ecotropic retroviral sequences highly related to Akv are present in some, but not all inbred mouse strains (19). In the wild, viruses with ecotropic host ranges have been almost exclusively obtained from mice from regions surrounding the Pacific (California, Japan, Southeast Asia) (20). Our results show that while an E-tropic-related retrovirus of the invention whose murine counterpart is rarely observed in mice and is known to infect only murides (rats or mice), it is infectious to canine cells.

The first possibility is pseudotyping. Mixed retroviral infections frequently exhibit pseudotyping, in which the genome of one virus is packaged in a virion containing SU proteins encoded by another virus. Pseudotyping could facilitate infection of cells not normally susceptible to infection (29).

However, the substitutions present in the envelope of a retrovirus of the invention are sufficient to enable this envelope to use the canine equivalent of the murine E-tropic receptor, resulting in an adaptation of the Akv virus to canine cells. Retroviral envelope proteins are composed of two subunits. The surface protein (SU) is responsible for receptor binding (38), and the transmembrane (TM) anchors the envelope proteins in the membrane and induces membrane fusion during virus entry. Sequence comparisons of MLV envelopes show regions of strong conservation, such as the C-terminal domain of TM, while others are variable like the N-terminal domain. There are two hypervariable regions, called VRA and VRB, and a recognizable proline-rich region in the SU proteins (Fig. 9).

Another nucleic acid molecule of the invention, less related to Akv Env is termed SU1. Previous studies have shown that P-tropic viruses are unable to infect canine cells (6). Using oligonucleotide primer pairs specific for the DLCV-1 SU1 VRA region, a retroviral sequence is amplified in canine cells but not in murine cells. However, fragments corresponding to X-tropic VRA are also amplified from canine cell lines, such as DLC 01 but are absent from D17 cells. These results suggest that the retroviral sequence corresponding to the VRA of DCLV-1 SU1 may be an endogenous retroviral sequence specific to canine cells. Furthemore this suggests that DCLV-1 virus has acquired a unique env sequence from an endogenous retroviral sequence after recombination with a competent ecotropic retrovirus such as Akv and/or P-tropic (probably defective) retroviral sequences. The divergence between a retrovirus of the invention and xenotropic retroviral sequences may be significant in terms of receptor usage since a specific deletion found in DLCV-1 SU1 sequence is situated at the major site of divergence between P-tropic and X-tropic viruses and corresponds to the main determinant for receptor selection as previously shown (1).

Nucleic acid molecules corresponding to the U3 region of a retrovirus of the invention are closely homologous to the mouse melanoma antigen. This antigen expression is associated in mice with transforming phenotypes. Nucleotide sequence analysis demonstrates that this antigen encodes the full length env gene and long terminal repeat region in an endogenous ecotropic Akv type murine leukemia provirus which is defective in C57BL/6 (17) .

The discovery of E-tropic retroviral envelope sequences in a cell of the invention as DLC 01 but not in another canine cell suggests that the replication competent retrovirus of the invention may be involved in leukemogenesis. The inbred laboratory mouse strain AKR displays a high incidence of spontaneous lymphoma and recombinant non-acutely transforming retroviruses are associated with the disease. The endogenous E-tropic viruses (inherited in AKR mice at two non-linked chromosomal loci, Akv-1 and Akv-2), the X-tropic virus and P-tropic viruses are found, but the proximal agents responsible for induction of leukemia in E-tropic virus infected mice are the P-tropic virus, generated by recombination events that involve endogenous proviral sequences in the mouse (16). After recombination, viruses utilize a cellular receptor different from that used by ecotropic MLVs and exhibit a broad host range.

Retroviruses of the invention that are well adapted to the host species may be innocuous for years after infection, or may induce hematopoietic malignancies after very long periods of latency. Endogenous retroviruses and retroviral elements represent a cellular reservoir of possible pathogenic retroviral genes able to recombine with E-tropic-competent retroviruses. However, the discovery of retroviral particles in a leukemic canine cell lines (T CD8⁺) and the presence of a RT activity in T-CD8⁺ tumoral cells suggests a possible relation between these CD8⁺ tumoral cells and retroviruses of the invention.
The env sequence of a retrovirus of the invention may confer original cell receptor interaction properties to said retrovirus.

### Figure legends

### Figure 1 :

### Dog lymphoma cells produce retroviral particles.

The DLC 01 cells were processed as described in materials and methods. Electron microscopy shows mature particles with a dense central core surrounded by a smooth envelope typical of type C retroviral particles. Arrows point thickening of the plasma membrane corresponding to site of assembly of the virions.

### Figure 2 :

### DLC 01 cells, and other T-lymphoproliferative diseased dogs produces Mn²⁺ dependant RT activity in their supernatant.

Dot blot of DNA polymerase activity. Viral particles purified from cell supernatant were monitored for RT activity, in presence of 6 mM MnCL2 (1, 2, 5 and 7), or 0.6 mM MgCl2 (3, 4, 7 to 9) using an exogenous poly(rA)-oligo(dT) primer template substrate and 32P-α-dTTP. Polymerization products were filtered on a DEAE 0,45-µm-pore-size membrane. Viral particles were purified from supernatant of DLC 01 (1 and 3), DLC 02 (2 and 4), CD8+ tumoral lymph node cells (5 and 8), and normal peripheral blood cells stimulated with IL2 and PHA (4 and 9) and culture medium used as negative control for Mn2⁺ (10).

Density gradient analysis of DCLV-1 viruses. Virus pelleted from cell supernatant were centrifugated through a 8 ml 20 to 60 % sucrose density gradient as described in material and methods. Each fractions of 250 µl encompassing the expected density peak of 1.17 g/l were monitored for the level of RT activity as in A and spot intensities were measured with a PhosphorImager. The analysis was repeated twice with similar results.

### Figure 3 :

### Cocultivation of different cell line with DLC 01 cells results in viral infection and spread.

D17, HeLa and Sup T1 cells were cocultivated with X-irradiated DLC 01 cells for 24 hours. After 2 weeks, 6 ml of supernatant from cocultivated cells or control parental cells were concentrated by centrifugation through a 20 % sucrose cushon. Each pellet was monitored for RT activity, using an poly(rA)-oligo(dT) substrate and 32T-α-dTTP. Polymerization products were filtered on a DEAE 0.45 µm-pore-size membrane, and spot intensity was measured with a PhosphorImager.

### Figure 4 :

### In vitro strong stop cDNA from DLCV-1 viral RNA.

Total RNA was purified from DLCV-1 virions and used as a substrate for in vitro reverse transcription using purified MoMuLV RT. The reaction products were loaded on a 6 % PAGE in presence of 7M urea and radiographied. A sequencing ladder was used for the lengh determination of the fragment.

RNA was purified using denaturating conditions, annealed with the 32P-labelled tRNApro oligonucleotide (see table 1), and reverse transcribed.

RNA was purified in non-denaturing conditions so that the endogenous tRNA annealed to the PBS could serve as a primer for in vitro reverse transcription in presence of 32P-α-dCTP.

### Figure 5 :

### DLCV-1 genomic RNA sequences amplified by RT-PCR.

On the top, a typical MLV RNA genome is represented with the coding sequences for all mature structural proteins. The positions of the sequence amplified from DLCV-1 and the oligonucleotides pairs used are indicated below. The annealing temperatures used for each oligonucleotide pair are the following :
- CMRT3180⁺/CMRT3280⁻ : 49°C ; CMPBS150⁺/CMCA1430⁻ : 53°C ;
- MLVSU6800⁺/MLVR8300⁻ : 58°C ; MLVRT3200⁺/MLVRT4250⁻: 60°C ;
- MLVIN5700⁺/MLVSU6880⁻ : 53°C ; MLVTM6800⁺/MLVR8300⁻: 58°C

### Figure 6 :

### Amplification of DLCV-1 genomic RNA by RT-PCR.

Virus were pelleted from DLC 01 cell supernatant through a 20 % sucrose cushon, RNA was purified, reverse transcribed and amplified using different oligonucleotides pairs. For A and B, the amplification products were eletrophoresed on an agarose gel and stained with ethidium bromide. For C, the amplification products were cloned in pGEM-T, DNA was prepared from several independent clones, digested with PvuII and eletroporesed on an agarose gel.

Amplification of the pol region using the CMRT3180⁺/CMRT3280⁻oligonucleotide pair. 1 : NIH/3T3 cells infected by F-MLV FB29 (positive control) ; 2 : DLCV-1 ; 3 : negative control without DNA ; 4 : DNA molecular weight marker.

Amplification of the env region using the MLVIN5700⁺/MLVSU6880⁻oligonucleotide pair. 1 : NIH/3T3 infected by F-MLV FB29 ; 2 : DLCV-1 ; 3 : negative control without DNA ; 4 : molecular weight marker.

PvuII analysis on individually cloned RT-PCR products of DLCV-1 viral RNA amplified with the MLVIN5700⁺/MLVSU6880⁻pair. Two PvuII sites are present in pGEM-T, one on each side of the cloning site. Lane 1, 2, 7 and 8 correspond to PCR products without PvuII site, lane 3, 4, 6 and 9 to PCR products with a PvuII site, lane 6 : in the reverse orientation when compared to the others. Lane 5 : undigested clone.

### Figure 7 :

### Alignment of different clones analysed and corresponding to pol region.

Nucleotides sequences and amino-acids translations.

### Figure 8 :

### Sequence alignment of the DLCV-1 clone with X-tropic and P-tropic MLVs.

The DLCV-1 SU1 sequence (indicated in bold) is compared with sequences representative of X-tropic and P-tropic MLVs. DBA2 Asc : DBA2 ascitis endogenous leukemia virus, NZB-9-1 : xenotropic endogenous virus, DLCV-1 : see text, MCF247 : Mink Cell Focus-inducing virus 247, MUSMLVPA : Polytropic endogenous sequence. Sequence divergences from DLCV-1 SU1 are indicated in shaded boxes. When DLCV-1 was divergent from all sequences at a position conserved among X-and P-tropic MLV, the divergence is shown as a shaded box on the DLCV-1 sequence. The Env initiation are boxed, in the MUSMLVPA endogenous retroviral sequence, the initiation codon was found to be mutated to ATA. The variable region VRA involved in the host range difference between X-tropic and P-tropic MLV is indicated. PMLVSUAUG⁺ and DLCVVRA⁻ primers used for genomic PCR amplification in figure 8 are shown.

### Figure 9 :

### Amino acid alignment derived from retroviral ENV SU proteins.

We compared aa with env of DCLV-1-SU9, x-tropic Bxv-1 and NZB-9-1, p-tropic MCF-247, E-tropic Akv, and DCLV-1-SU9. DVLV-1 is used as the standard. Dots show identity. Variables regions VRA, VRB and PRR are indicated.

### Figure 10 :

### Presence of DCLV-1-related sequences in the genome of dogs.

Cells were lysed as described in materials and methods and genomic DNA was extracted, and PCR amplified.

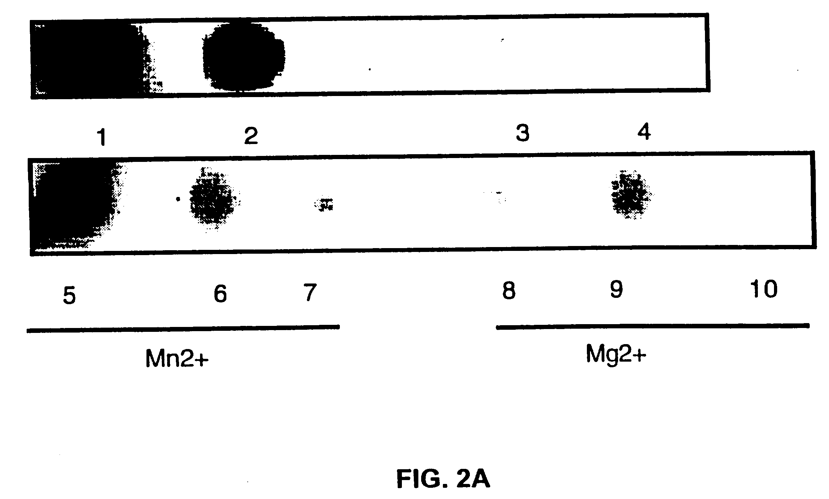

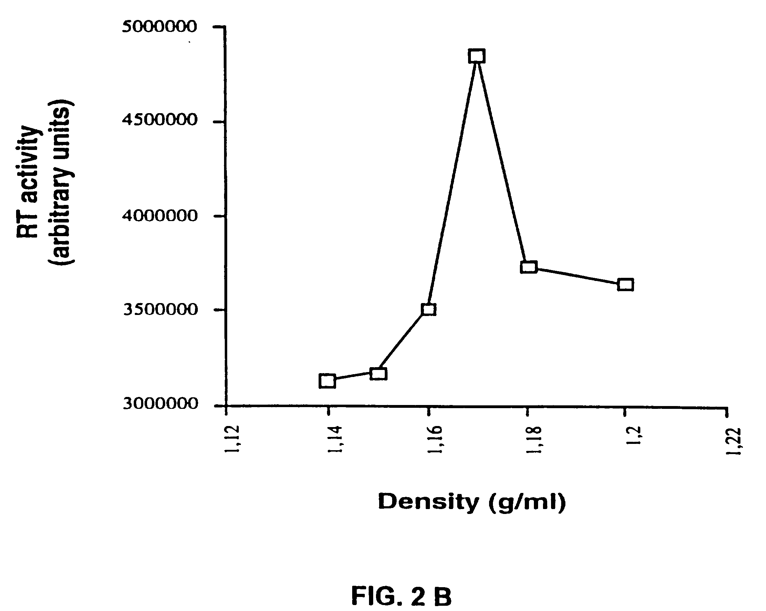

Amplification of the pol region using the CMRTR3180⁺/CMRT3280⁻oligonucleotide pair. Molecular weight marker. 2 : NIH/3T3 infected with MLV FB29. 3, 4 : PBMC from healthy dogs. 5 : DH82 cell line. 6 : D17 cell line. 7 : CD8⁺ dog tumoral lymph node cells (see figure 2). 8 : DLC 02 dog cell line. 9 : DLC 01 cell line. 10 : without DNA.

Amplification of the env region using the PMLVSUATG⁺/DLCVVRA⁻oligonucleotide pair. 1 : CD8⁺ dog tumoral lymph node cells (see figure 2). 2 : D17 cell line. 3 : DH82 cell line. 4 : NIH/3T3 cell line. 5 : without DN. 6 : molecular weight marker. PCR was performed as described in material and methods an annealing temperature of 58°C.

### Figure 11 :

### Oligonucleotide primers and target sites.

a) Oligonucleotides where names strat with CM and MLV were designed in order to anneal to sequences conserved among Mammalian type C oncoviruses and among Murine leukemia viruses respectively ;
b) with respect to the Akv complete genome (MLOCG)
c) na : non applicable (sequences not found in the Akv genome)

### Figure 12 :

### Homologies between Akv-related DLCV-1 proteins sequences and E-tropic murine leukemia virus.

A : correspond to CA-encoding region,
b : correspond to PR and RT-encoding regions.

### Examples

### Example 1 : Electron microscopy

After induction with 0.5% DMSO for 1 day, cells are centrifuged at 400 x g for 10 min and resuspended. Cell suspension (106 cells) is fixed in 2% glutaraldehyde and 0.5% paraformaldehyde in 0.1 M phosphate buffer, then post-fixed in 1 % Os04. After alcoholic dehydratation, samples are embedded in epoxy resin, and stained ultrathin sections of samples are examined by electron microscopy at the CMEABG (Centre de Microscopie Electronique Appliquée à la Biologie et à la Géologie, Villeurbanne, France) with a CM120 apparatus (Philips, Holland).

The morphology of the particles observed by electron microsocopy in the supernatant of DLC 01 cells is similar to a type C retrovirus (Figure 1). As previously mentioned, the virions originated from highly stained areas at the cell membrane, indicating that capsid assembly is concommitant with budding. Both mature and immature particles can be observed. Mature particles exhibit a central dense core, separated from the envelope by an electron-lucent space, and envelope spikes can not be clearly seen.

### Example 2 : Sucrose density gradient analysis

Virus particles are layered onto 8 ml 20 to 60% sucrose gradients prepared in 10 mM Tris-HCl (pH 7.5)-100 mM NaCl. Gradients are centrifuged in an SW41 rotor at 80,000 x g for 18 hr at 4°C. 250 µl fractions are carefully collected from the bottom, using a Pasteur pipette connected to a peristaltic pump. Density is calculated on 10 fractions from each gradient by using a refractometer ; density of the other fractions is extrapolated from the linear curve obtained. Ten µl from each fraction are used to quantitate RT activity.

### Example 3 : Reverse transcriptase (RT) assay.

To confirm the retroviral origin of the virus particles observed by electron microscopy, the supernatant of DLC 01 cells are tested for reverse transcriptase (RT) activity :

Viruses from 18-h harvests are clarified by filtration using 0,45-µm-pore-size filters and concentrated through a RNase-free 25% sucrose cushion by centrifugation at 30000 rpm for 3 hr (Beckman, TI 45). The pelleted material is resuspended in TNE buffer (150mM NaCl, 10mM Tris ph8, 1mM EDTA), and used for RT assays.

Reverse transcriptase assays are routinely performed according to published procedures (13). RT is measured by the incorporation of thymidine from 32P-dTTP (1Ci/mM) into a poly (rA): oligo dT(15) primer-template substrate in buffer containing 50 mM Tris-Cl, pH 8.3, 20 mM DTT, 0.25% Nonidet P40, and either 6 mM MgCl2 or 0.6 mM MnCl2. After 1 hour incubation at 37°C, samples are dot-blotted on a DEAE 0.45-µm-pore-size membrane using a Biorad dot-blotter apparatus, extensively washed with 3 M NaCl, 0.3 M Na Citrate, pH 7.0, and spot intensity is measured with a PhosphoImager. A QT6 (Quail Embryonic fibroblast) cell line infected by Rous sarcoma virus (RSV) and a NIH/3T3 cell line infected by Friend murine leukemia virus strain 57 were used as positive controls for Mg²⁺ dependent and Mn²⁺ dependent RT activity, respectively.

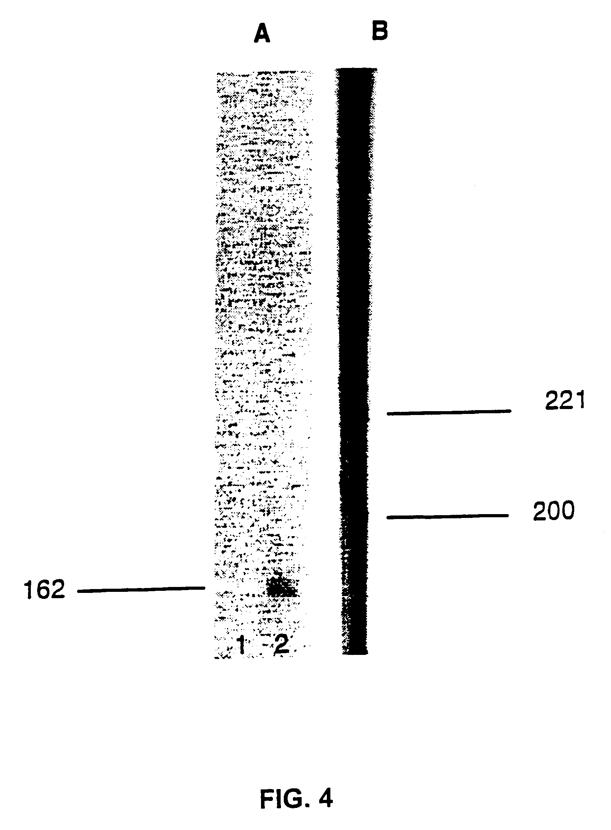

When compared to peripheral blood mononuclear cells from an healthy dog as a negative control, DLC 01 supernatant is clearly positive for RT activity (compare spots 1 and 6 in Figure 2A). Two other samples are also tested : a second canine T cell line derived from a large granular CD8⁺ T-cell leukemia and primary CD8+ T cells collected from a tumoral lymph node of a third dog. Both of these supernatants also contain a RT activity (Figure 2A, spots 2 and 5). As shown in figure 2B, the DLC 01 RT activity is not free in the supernatant but sediments in a sucrose gradient at a density of 1.17 g/ml, typical for retroviral particles. The DCLV-1 RT activity shows a marked preference for Mn2⁺ over Mg2⁺ as cofactor (compare Figure 2, spot 1 to 4). These characteristics are suggestive of a mammalian type C oncovirus. To confirm this hypothesis, we perform an in vitro reverse transcription assay using purified Mo-MuLV RT and RNA purified from virion. When the reaction is primed with a DNA oligonuclotide corresponding to the eighteen 3'-terminal nucleotides of tRNApro (see Figure 11, tRNA^{pro}), a 162 nucleotide product is obtained (Figure 4A) indicating that DLCV-1 uses tRNApro as a primer for the initiation of minus strand DNA synthesis, and that its R-U5 region is 144 nucleotides long (162-18). This observation is confirmed in a reaction using the endogenous tRNA as a primer. This reaction gives a minus strand stong-stop product of 221 nucleotides corresponding to the 144 nt long R-U5 region plus the 75 nt-long tRNApro (Figure 4B). The only known retroviruses using primer tRNA^{pro} are mammalian type C oncovirus and type C viruses from the HTLV/BLV group. However, the latter have a longer R-U5 region and a Mg²⁺-dependent RT activity.
Thus, DLCV-1 is a mammalian type C oncovirus.

### Example 4 : In vitro strong-stop cDNA synthesis

### 1°/ tRNA-Primed reverse transcription.

Reverse transcription was performed using 10 UI of MoMLV RT in 30 mM Tris (pH8.2), 3 mM MgCl2, 50 mM NaCl, 10 mM DTT, 100 ng purified RNA, 0.25 mM dATP, dGTP and dTTP) and 0.25 µM dCTP, with 2 µCi of -32P)-dCTP (Amersham) per reaction for 20 min at 37° C.

### 2°/ 32P-labelled oligonucleotides primers.

RT was performed as previously described (8), using 100 ng of template and 0.4 pmol primer. Primer-template annealing was performed by heat (3 min at 95°C then 30 min at 55°C). Primer was an 18 nt DNA oligonucleotide complementary to the PBS (Primer Binding Site). MoMLV RT (100 UI) was added with dNTPs (0.25 mM each) and synthesis was for 20 min in 30 mM Tris-HCl (pH 8.2), 3 mM MgCl2, 50 mM NaCl, 10 mM DTT in a 10 µl volume.

For each experiment, the polymerisation was stopped by addition of 1% (w/v) SDS, 40 mM EDTA and incubated for three minutes at 65°C, and proteins were removed by extraction with phenol and phenol/chloroform. Nucleic acids were precipitated with three volumes of ethanol, dissolved in 95% (v/v) formamide, heat denatured 3 minutes at 95° C and resolved by 6% PAGE in 7 M urea and 50 mM Tris-borate-EDTA (pH 8.3).

### Example 5 : Infections by coculture

The DLC 01, a T CD⁸⁺ dog leukemic cell line, a CD⁸⁺ tumoral dog cells (isolated from a dog suffering from a cutaneous lymphoma) and human sup T1 cell line are grown in RPMI 1640 culture medium (Cergy Pontoise, France), supplemented with 10%-20% heat-inactivated fetal calf serum (FCS) (Bohenger), DMEM complemented with 10% NCBS for NIH cell line or 10%FCS for D17, L-glutamine (2 mM), sodium pyruvate (2mM), penicillin (100 IU/mL) and streptomycin (100 µg/mL), at 37° C in an atmosphere containing 5% C02.

For infection by coculture, 10⁶ cells from the producer cell line DLC 01 are X ray-irradiated (6000 rad), mixed with 2x10⁵ target cells, NIH/3T3, D17, SupT1 and HeLa, and passaged every 3-4 days. Infection is monitored after 15 days and then at weekly intervals by analysis of RT activity of the virus pellet from 6 ml of supernatant (see example 2).

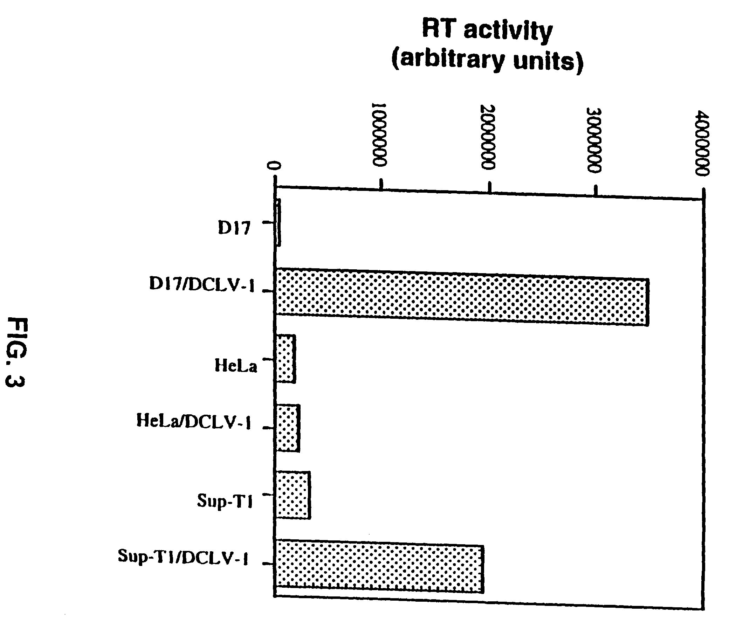

In order to investigate infectivity of these virions, irradiated DLC 01 cells are cocultivated with various recipient cells which are of canine (D17 osteosarcoma cells), human (Hela carcinoma and SupTl T lymphoid cells) and murine (NIH/3T3 fibroblast cells) origin. One week after irradiation, we can not detect any viable cells in control irradiated DLC 01 cells and the corresponding supernatant is negative for RT activity. In contrast, three weeks after cocultivation, D17 canine cells, but not a D17 negative control, produce high levels of RT activity in the supernatant, demonstrating that the DLC 01 cell line produces retroviruses able to infect canine cells (Figure 3). Similarly, the SupT1 cells are infected after cocultivation, but RT activity in the cocultivated Hela cell supernatant is not significantly different from the background in the negative control Hela cells. We can also infect by cocultivation NIH/3T3 cells. Thus DLC 01 cells produce an infectious retrovirus with a broad host range.

### Example 6 : cloning and sequencing of the particles collected from the supernatant of DLC 01 cells

### 1°/ RNA extraction and cDNA synthesis

Viruses from 18-h harvests are clarified by filtration using 0,45-µm-pore-size filters and concentrated through a RNase-free 25% sucrose cushion by centrifugation at 30000 rpm for 3 hr (Beckman, TI 45). The pelleted material is resuspended in TNE buffer (150mM NaCl, 10mM Tris ph8, 1mM EDTA), and used for RNA extraction.

Total RNA from particles collected from the supernatant of DLC 01 cells and concentrated as above is extracted using trizol (Life technologies) under conditions recommended by the manufacturer. Pellets are resuspended in DNase buffer (40mM Tris pH = 7.5, 6mM MgCl2, 10mM NaCl, 10 mM dithiothreitol, 200 U/ml RNasin per ml). Contaminant DNA are digested with RQ1 DNase (100U/ml) (Promega) for 15 min at 37°C, and proteins removed by phenol and phenol/chloroform extractions. After ethanol precipitation, 1 µg of total viral RNA is dissolved in 15 µl of DEPC-treated water and denatured at 95° for 2 min. After incubation on ice for 1 min, 6 µl of 5xRT buffer (250 mM Tris-HCL (pH 8.3), 375 mM KCL, 15 mM MgCl2), 6 µl 2.5 mM dNTPs, 1 µl RNasin (Ribonuclease Inhibitor) (Promega) and 200 UI of Moloney murine leukemia virus (M-MLV) reverse transcriptase (Life technologies) are added. cDNA synthesis is performed at room temperature for 10 min, at 37°C for 50 min and denatured at 95 °C for 5 min.

### 2°/ DNA oligonucleotide primers and PCR analysis.

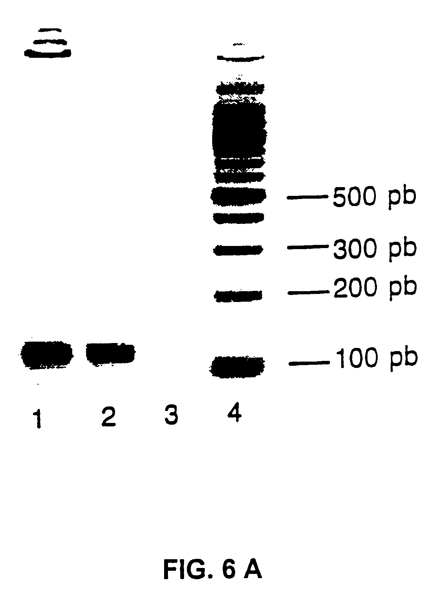

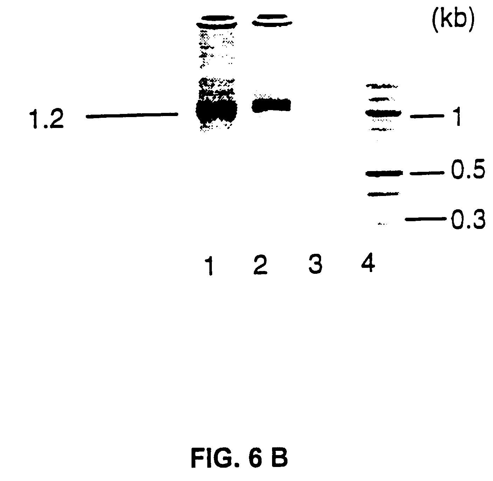

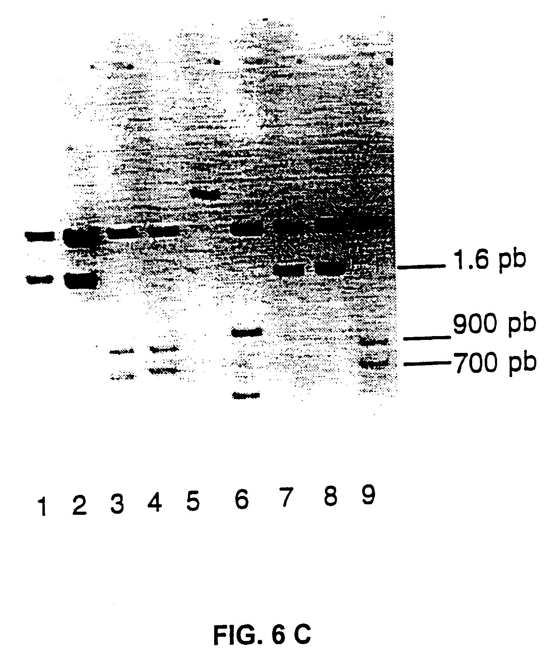

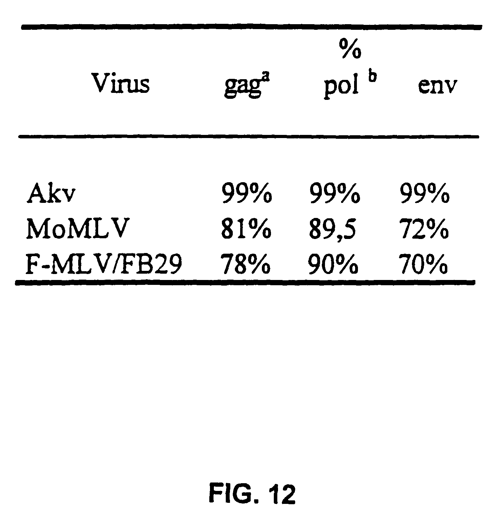

Oligonucleotides primers and target sites used are shown in Figure 11 and numbered according to AKV sequence (figure 6). PCR is performed with 10 µl of cDNA reaction mixture or 0.5 µg genomic DNA and 50 pmol of each primer. The reaction mixture consistly 50 µl, with 2.5 mM MgCl2, 50 mM KCL, 10 mM Tris-HCL (pH 9), 0.1% triton X-100, 50 pmol of each oligonucleotide, 0.2 mM each deoxynucleoside triphosphate, and 1 UI of Promega Taq polymerase. After a hot start, the amplification cycle was as follows : 35 cycles (94°C for 1 min, 55°C (from 49 to 58°C), (depending primers used; hybridation temperatures for each primer pair are indicated in Figure 12) for 3 min, 72°C for 5 min) and incubated at 72°C for 8 min for a final extension. The reaction products are then electrophoresed on 1.5% agarose gels, and detected by ethidium bromide staining.

Cells for genomic DNA amplification are lysed in 10 mM Tris (pH 8), 100 mM NaCl, 10 mM EDTA, 1% SDS, treated with 100 µg/ml of RNase for one hour at 37°C and incubated 3 hours at 50°C with 100 µg/ml of proteinase K. Proteins are extracted twice with phenolchloroform and once with chloroform, and total DNA is ethanol precipitated and resuspended in 500 µl of water. 0.5 µg of genomic DNA is used to perform PCR as described.

### 3°/ Cloning and sequencing

PCR generates DNA products which are electrophoresed on 1.3% agarose gels, and products are excised, purified using Jet sorb kit (GENOMED), as recommended by the manufacturer and cloned into the PGEM-T vector (Promega). Cloned inserts are sequenced in both directions using SP6 and T7 primers, except for the MLVIN 5700⁺/MLVSU6880⁻ amplification product which uses the MLVSU7100⁺ internal primer by Eurogentec or manually with the Sequenase kit (ABI PRISM).

### 4°/ Sequence alignment

Retroviral sequences are initially identified by BLAST searches. Nucleotide sequence alignments are made with local align program. Other MLV sequences are obtained from GenBank.

### 5°/ Results of the sequence analysis

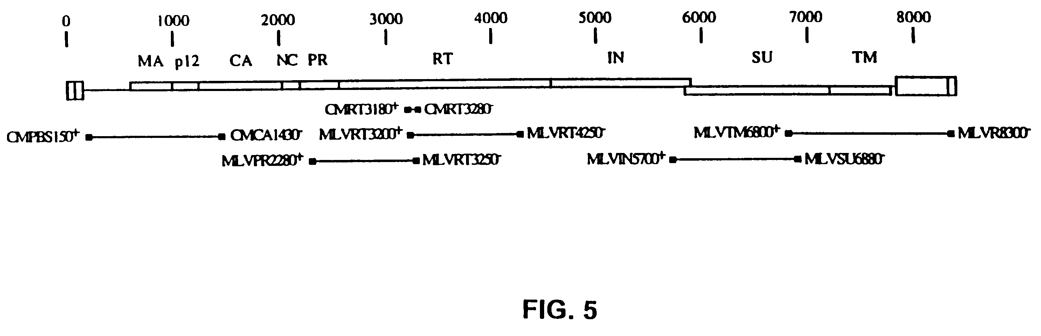

- DLCV-1 is highly related to murine leukemia viruses The identification of DLCV-1 as a mammalian type C oncovirus allows us to design two sets of degenerate primers which anneal to regions conserved in all members of this genus (CMRT3180⁺/CMRT3280⁻ and CMPBS150⁺/CMCA1430⁻, see Figure 11 and Figure 5) and to amplify by RT-PCR a small part of the RT-encoding region (Figure 6A) and a 5'leader/5'gag region from DLCV-1 genomic RNA.
   The RT-derived PCR fragment is cloned, and several clones are sequenced. All of them turn out to be closely related to MLV sequences, however there are several divergent nucleotides, in one case resulting in an amino-acid substitution (Figure 7).
   This indicates that the DCLV-1 virions are derived from several independant viral sequences.
   The 5'leader/5'gag fragments are also cloned and sequenced. These fragments have been found to be closely related to MLV sequences.
   Since DCLV-1 appears to be homologous to MLV in several regions, two additonal primer pairs which recognized sequences conserved among MLV strainshave been used to amplify, clone and sequence about 2 Kb within the PR/RT-encoding region (MLVPR2280⁺/MLVRT3250⁻ and MLVRT3200⁺/MLVRT4250⁻, see Figure 11 and Figure 5).
   As shown in Figure 12, DLCV-1 is particularly homologous to Akv, a T lymphoma-inducing MLV spontaneously produced from retroviral endogenous sequences in AKR mice, with about 99 % homology at the nucleotide level in all the regions amplified. The nucleotide sequences appear to be much more conserved between DLCV-1 and Akv than between Akv and other MLVs, such as Friend-MLV (F-MLV) or Moloney-MLV (M-MLV) (Figure 12).
   Several divergences with Akv in the coding sequences resulted in a few amino acid substitutions. However, none of them affected aminoacids known to have a particular functional importance.
- the DLCV-1 viruses show sequence heterogeneity in the env- and U3 regions.
   The env and U3 regions of MLV are more divergent from strain to strain than other parts of the genome, due in large part to the existence of envelopes which interact with different cell receptors and therefore confer various host ranges to the strains harboring them. Additionally, there is variability in the length, number of transcriptional enhancer sequences within the U3 region, controlling cell-type tropism, transcriptional efficiency and pathogenicity of the viruses (31), (32), (33). Two primer pairs (MLVIN5700⁺/MLVSU6880⁻ and MLVSU6800⁺/MLVR8300⁻, see Figure 11 and Figure 5) that recognize sequences conserved among all MLV strains and allow the amplification of the whole env and U3 regions of DLCV-1 (Figure 6B) have been designed. While the amplification products obtained with both primer pairs are again closely related to MLV sequences, they appear to be more divergeant from Akv and more heterogenous than the regions previously amplified (see above).
   Moreover, restriction analysis of cloned RT-PCR fragments encoding the N-terminal part of the SU subunit distinguishes two sets of sequences that differ by the presence or absence of a PvuII site (Figure 6C).
   Sequences without the PvuII site are highly related to Akv (about 99 % homology at the nucleotide level, Figure 12), an E-tropic (ecotropic) virus known to infect only murine cells.
   PvuII-bearing sequences are less related to known MLV sequences and more homologous to X-tropic (xenotropic) viruses (that show a broad host ranges, including dogs) or to P-tropic (polytropic) viruses than to E-tropic viruses.
   Partial sequence alignment of a PvuII-bearing clone (DLCV-1 SU1) with four representative X-tropic or P-tropic sequences is shown in Figure 8. Further examination of this alignment presents several interesting features. First, this clone appears to be a recombinant between P-tropic and an X-tropic sequences. In the region of the Env initiation codon, it is clearly related to P-tropic envelopes. There is a 12-base-pair insertion immediately prior to the ATG codon also found in P-tropic sequences, and only one substitution within the 100 first nucleotides of the alignment, at a position conserved between P- and X-tropic viruses. In contrast, in the remainder of the alignment, DLCV-1 SU-1 is much more closely related to X-tropic than to P-tropic viruses. Second, even within this X-tropic related region, DLCV-1 SU-1 is much more divergent from both the X-tropic sequences than these sequences are from each other. A multiple alignment including several other X-tropic sequences confirm this observation. Strikingly, DLCV-1 SU-1 contains a 9-base pair deletion (positions 330-338) and additional surrounding substitutions which are not found in any X-tropic sequence. Using a primer specific for this region, we are unable to amplify any endogenous sequence from murine cells (Figure 10B). This deletion is situated at the major site of divergence between P and X-tropic viruses, within the variable region A (VRA) known to be responsible for host range differences in between these two types of viruses, and suggests that it could confer original cell receptor interaction properties to the DLCV-1 envelope.
   The U3 region is shorter than that of Akv. DCLV-1 U3 region is close to mouse melanoma antigen with only 2 aminoacids substitution. Nucleotide sequence are similar to Akv among U3, but contain a 100 bp deletion.
   The complete amino-acid sequences of DLCV-1 SU9 (an Akv-related env sequence) and SU1 are compared with envelope sequences from different host-range classes of MLV. As shown in Figure 10, SU9 presents only 4 substitutions, when compared to the E-tropic Akv. Three of them (S181L, E186K, T196A) are situated between the two variable regions VRA and VRB involved in receptor recognition, and the fourth (T238I) is at the beginning of VRB. Similarly, except for a divergence which results in 4 consecutive amino-acid changes, SU1 presents about 12 substitutions when compared to NZB-9-1 or Bxv-1 xenotropic sequences; all of them are conservative.
- Sequences related to the DLCV-1 specific envelope are present in the canine genome.
   In order to examine whether this sequence is unique to DLCV-1 and to identify a possible origin of this virus, PCR analysis on genomic DNA from canine and murine cells has been performed. The CMRT3180⁺/CMRT3280⁻ primer pair, which allows the amplification of sequences homologous to a small portion of the pol region of any type C mammalian oncovirus (Fig 10A) has first been used.
   High yields of a 114 bp specific product have been produced from an NIH/3T3 cell line chronically infected with F-MLV strain FB29 (lane 2), and the DLC 01 cell line as well as the RT-producing DLC-02 dog cell line (lane 8 and 9) (see figure 2). The RT-producing primary T-lymphoma cells give a lower yield of this product (lane 7).
   A specific product has interestingly been obtained from the genomic DNA of two healthy dogs (lane 3 and 4) and from uninfected canine cell lines (D17 and DH82) (lane 5 and 6) indicating that pol-related sequences are present in the normal canine genome. The 114 pb PCR product from the D17 and DLC 02 cell lines have been cloned and sequenced. They are highly homologous to the Akv sequence (data not shown).
   Moreover, using a primer pair (PMLVSUAUG+/DLCVVRA-) specific for the DLCV-1 SU1 ENV sequence (see Figure 8), a 300 pb specific sequence present in the D17 and DH82 dog cell lines, but absent from NIH/3T3 (figure 10B) has been amplified.
   Therefore, DLCV-1-SU1-related endogenous sequences are present in the canine genome but not in the murine genome. The DLCV-1-SU1 virus appears to be related to endogenous retroviral sequences.

### REFERENCES

1. Battini, J. L., P. Rodrigues, R. Muller, O. Danos, and J. M. Heard. 1996. Receptor-binding properties of a purified fragment of the 4070A amphotropic murine leukemia virus envelope glycoprotein. J Virol. 70:4387-93.
2. Benton, C. V., B. L. Brown, J. S. Harshman, and R. V. Gilden. 1981. In vitro host range of equine infectious anemia virus. Intervirology. 16:225-32.
3. Bonham, L., G. Wolgamot, and A. D. Miller. 1997. Molecular cloning of Mus dunni endogenous virus: an unusual retrovirus in a new murine viral interference group with a wide host range. J Virol. 71:4663-70.
4. Bouillant, A. M., G. M. Ruckerbauer, and K. H. Nielsen. 1989. Replication of the bovine immunodeficiency-like virus in diploid and aneuploid cells: permanent, latent and virus-productive infections in vitro. Res Virol. 140:511-29.
5. Chapman, A. L., W. J. Bopp, A. S. Brightwell, H. Cohen, A. H. Nielsen, C. R. Gravelle, and A. A. Werder.
1967. Preliminary report on virus-like particles in canine leukemia and derived cell cultures. Cancer Res. 27:18-25.
6. Cloyd, M. W., M. M. Thompson, and J. W. Hartley.
1985. Host range of mink cell focus-inducing viruses. Virology. 140:239-48.
7. Coffin, J. M., S. H. Hughes, and H. E. Varmus.
1997. Retroviruses, 3rd ed ed, vol. 1. Cold Spring Harbor Laboratory Press, New York.
8. Darlix, J. L., C. Gabus, and B. Allain. 1992. Analytical study of avian reticuloendotheliosis virus dimeric RNA generated in vivo and in vitro. J Virol. 66:7245-52.
9. Francki, R., C. Fauquet, D. Knudson, and F. Brown. 1991. Classification and nomenclature of viruses. Fifth report of the international committee and toxonomy of viruses. Springer-Verlag, New York.
10. Gardner, M. B., B. E. Henderson, J. D. Estes, H. Menck, J. C. Parker, and R. J. Huebner. 1973. Unusually high incidence of spontaneous lymphomas in wild house mice. J Natl Cancer Inst. 50:1571-9.
11. Gardner, M. B., B. E. Henderson, J. E. Officer, R. W. Rongey, J. C. Parker, C. Oliver, J. D. Estes, and R. J. Huebner. 1973. A spontaneous lower motor neuron disease apparently caused by indigenous type-C RNA virus in wild mice. J Natl Cancer Inst. 51:1243-54.
12. Ghernati, I. Auger, C. Chabanne, L. Corbin, A. Bonnefont, C. Magnol, J. P. Fournel, C. Rivoire, A. Monier, J. C. Rigal, D. Characterization of a Canine Long-term T-cell Line (DLC 01) Established from a Dog with a Sézary Syndrome and producing retroviral particles. leukemia, in press.
13. Goff, S., P. Traktman, and D. Baltimore. 1981. Isolation and properties of Moloney murine leukemia virus mutants: use of a rapid assay for release of virion reverse transcriptase. J Virol. 38:239-48.
14. Graves, D. C., and J. F. Ferrer. 1976. In vitro transmission and propagation of the bovine leukemia virus in monolayer cell cultures. Cancer Res. 36 :4152-9.
15. Gross, L. 1951. Spontaneous leukemia developing in C3H mice following inoculation in infancy, with A-K leukemic extracts, or A-K embryos. Proc. Soc. Exp. Biol. Med. 76:27-32.
16. Hartley, J. W., N. K. Wolford, L. J. Old, and W. P. Rowe. 1977. A new class of murine leukemia virus associated with development of spontaneous lymphomas. Proc Natl Acad Sci U S A. 74:789-92.
17. Hayashi, H., H. Matsubara, T. Yokota, I. Kuwabara, M. Kanno, H. Koseki, K. Isono, T. Asano, and M. Taniguchi. 1992. Molecular cloning and characterization of the gene encoding mouse melanoma antigen by cDNA library transfection. J Immunol. 149:1223-9.
18. Herniou, E., J. Martin, K. Miller, J. Cook, M. Wilkinson, and M. Tristem. 1998. Retroviral diversity and distribution in vertebrates. J Virol. 72:5955-66.
19. Jenkins, N. A., N. G. Copeland, B. A. Taylor, and B. K. Lee. 1982. Organization, distribution, and stability of endogenous ecotropic murine leukemia virus DNA sequences in chromosomes of Mus musculus. J Virol. 43:26-36.
20. Kozak, C. A., and R. R. O'Neill. 1987. Diverse wild mouse origins of xenotropic, mink cell focus-forming, and two types of ecotropic proviral genes. J Virol. 61:3082-8.
21. Lavelle, G., L. Foote, R. L. Heberling, and S. S. Kalter. 1979. Expression of baboon endogenous virus in exogenously infected baboon cells. J Virol. 30:390-3.
22. Martin, J., E. Herniou, J. Cook, R. W. O'Neill, and M. Tristem. 1999. Interclass transmission and phyletic host tracking in murine leukemia virus-related retroviruses. J Virol. 73:2442-9.
23. Modiano, J. F., D. M. Getzy, K. G. Akol, T. J. Van Winkle, and G. L. Cockerell. 1995. Retrovirus-like activity in an immunosuppressed dog: pathological and immunological findings. J Comp Pathol. 112:165-83.
24. Okuma, K., M. Nakamura, S. Nakano, Y. Niho, and Y. Matsuura. 1999. Host range of human T-cell leukemia virus type I analyzed by a cell fusion-dependent reporter gene activation assay. Virology. 254:235-44.
25. Onions, D. 1980. RNA-dependent DNA polymerase activity in canine lymphosarcoma. Eur J Cancer. 16:345-50.
26. Rein, A., and A. Schultz. 1984. Different recombinant murine leukemia viruses use different cell surface receptors. Virology. 136:144-52.
27. Riedel, N., E. A. Hoover, R. E. Dornsife, and J. I. Mullins. 1988. Pathogenic and host range determinants of the feline aplastic anemia retrovirus. Proc Natl Acad Sci U S A. 85:2758-62.
28. Safran, N., K. Perk, 0. Eyal, and J. E. Dahlberg.
1992. Isolation and preliminary characterisation of a novel retrovirus isolated from a leukaemic dog. Res Vet Sci. 52:250-5.
29. Sitbon, M., J. Nishio, K. Wehrly, and B. Chesebro. 1985. Pseudotyping of dual-tropic recombinant viruses generated by infection of mice with different ecotropic murine leukemia viruses. Virology. 140:144-51.
30. Sommerfelt, M. A., and R. A. Weiss. 1990. Receptor interference groups of 20 retroviruses plating on human cells. Virology. 176:58-69.
31. Speck, N. A., and D. Baltimore. 1987. Six distinct nuclear factors interact with the 75-base-pair repeat of the Moloney murine leukemia virus enhancer. Mol Cell Biol. 7:1101-10.
32. Speck, N. A., B. Renjifo, E. Golemis, T. N. Fredrickson, J. W. Hartley, and N. Hopkins. 1990. Mutation of the core or adjacent LVb elements of the Moloney murine leukemia virus enhancer alters disease specificity. Genes Dev. 4:233-42.
33. Speck, N. A., B. Renjifo, and N. Hopkins. 1990. Point mutations in the Moloney murine leukemia virus enhancer identify a lymphoid-specific viral core motif and 1,3-phorbol myristate acetate- inducible element. J Virol. 64:543-50.
34. Stoye, J. P., and J. M. Coffin. 1987. The four classes of endogenous murine leukemia virus: structural relationships and potential for recombination. J Virol. 61:2659-69.
35. Takeuchi, Y., C. Patience, S. Magre, R. A. Weiss, P. T. Banerjee, P. Le Tissier, and J. P. Stoye. 1998. Host range and interference studies of three classes of pig endogenous retrovirus. J Virol. 72:9986-91.
36. Tomley, F. M., S. J. Armstrong, B. W. Mahy, and L. N. Owen. 1983. Reverse transcriptase activity and particles of retroviral density in cultured canine lymphosarcoma supernatants. Br J Cancer. 47:277-84.
37. Varnier, 0. E., C. M. Repetto, S. P. Raffanti, A. Alama, and J. A. Levy. 1983. Host range differences among xenotropic type C retroviruses isolated from mouse kidney cell cultures. J Gen Virol. 64:425-8.
38. Weiss, R. A. 1993. Cellular receptors and viral glycoproteins involved in retrovirus entry, p. 1-108. In L. J. A. (ed) (ed.), The Retroviridae, vol. Plenum Press, New York, N. Y.

## Claims

1. Retrovirus characterized in that :
- it is of type C morphology ;
- it sediments in a sucrose gradient at a density comprised between 1.16 g/l and 1.18 g/l ;
- it is infectious for dog cells ; and
- it belongs to the oncovirinae group.

2. Retrovirus according to claim 1 characterized in that : the dog cells are chosen among dog lymphocytes, CD8⁺ tumoral dog cells, dog cells isolated from a dog suffering from a cutaneous lymphoma, DLC 01 cells (registered at the CNCM the 17^{th} May, 1999 under registry number I-2200) and/or D17 dog cells.

3. Retrovirus according to claim 1 or 2 characterized in that : it belongs to the mammalian type C oncovirus group.

4. Retrovirus according to any one of claims 1 to 3 characterized in that : it is a spherical particle of about 100 nm in diameter.

5. Retrovirus according to any one of claims 1 to 4 characterized in that : it is produced by the canine DLC 01 cell line deposited at the CNCM the 17^{th} May 1999 under registry number I-2200.

6. Retrovirus according to any one of claims 1 to 5 characterized in that : it is infectious for mammalian cells.

7. Retrovirus according to claim 6 characterized in that : mammalian cells are murine or human cells.

8. Retrovirus according to any one of claims 1 to 7 characterized in that : it encodes an Mn2⁺ dependent reverse transcriptase.

9. Retrovirus according to any one of claims 1 to 8 characterized in that : it uses the tRNA^{pro} for the initiation of reverse transcription.

10. Retrovirus according to any one of claims 1 to 9 characterized in that : it is highly homologous to a Murine Leukemia Virus (MLV) in regards with its nucleotide sequence and/or amino acid sequence.

11. Retrovirus according to claim 10 characterized in that : the MLV virus is chosen among an E-tropic, a P-tropic, a X-tropic or an A-tropic MLV virus.

12. Retrovirus according to any one of claims 1 to 11 characterized in that : it comprises a gag region which is highly homologous to the gag region of an MLV virus.

13. Retrovirus according to any one of claims 1 to 12 characterized in that : it comprises a pol region which is highly homologous to the pol region of an MLV virus.

14. Retrovirus according to any one of claims 1 to 13 characterized in that : it comprises a pol region whose cDNA comprises at least one of the DNA sequences depicted in figure 7.

15. Retrovirus according to any one of claims 1 to 14 characterized in that : it comprises an env region which is highly homologous to the env region of an MLV virus.

16. Retrovirus according to any one of claims 1 to 15 characterized in that : it comprises a SU region which encodes at least part of the DCLV-1-SU9 amino acid sequence depicted in figure 9, said part comprising at least one of the substitution or deletion which distinguishes DCLV-1-SU9 from Akv as depicted in figure 9.

17. Retrovirus according to any one of claims 1 to 16 characterized in that : it comprises a SU region which encodes at least part of the DCLV-1-SU1 amino acid sequence depicted in figure 9, said part comprising at least one of the substitution or deletion which distinguishes DCLV-1-SU1 from the Bxv-1, and/or from NZB-9-1 and/or from MCF247 sequences depicted in figure 9.

18. Retrovirus according to any one of claims 1 to 17 characterized in that : it comprises a SU region whose cDNA comprises at least part of the DCLV-1-SU1 nucleotide sequence depicted in figure 8, said part comprising at least one of the substitution or deletion which distinguishes DCLV-1-SU1 from DBA2 asc and/or from NZB-9-1 and/or from MCF247 and/or from MUSMLPVA sequences depicted in figure 8.

19. Retrovirus according to any one of claims 1 to 18 characterized in that : it is an endogenous retrovirus.

20. Retrovirus according to any one of claims 1 to 19 characterized in that : it is able to immortalize dog cells.

21. Retrovirus according to any one of claims 1 to 20 characterized in that : it is isolated from the supernatant of DLC 01 cells registered at the CNCM the 17^{th} may 1999 under registry number I-2200.

22. Sub-unit characterized in that : it can be derived or isolated from a retrovirus according to any one of claims 1 to 21.

23. Sub-unit characterized in that : it can be cloned or sequenced from a retrovirus according to any one of claims 1 to 21.

24. Sub-unit according to claim 22 or 23 characterized in that : it is a nucleic acid molecule or a polypeptide.

25. Nucleic acid molecule, eventually according to claim 24 characterized in that : it comprises in its sequence a sequence encoding a gag region or a part thereof which is highly homologous to the gag region of an MLV virus or to a part thereof.

26. Nucleic acid molecule according to claim 24 or 25 characterized in that : it comprises in its sequence a sequence encoding an amino acid sequence of a pol region or a part thereof which is highly homologous to the pol region of an MLV virus or to a part thereof.

27. Nucleic acid molecule according to any one of claims 24 to 26 characterized in that : it comprises in its sequence a sequence encoding an env region or a part thereof which is highly homologous to the env region of an MLV virus.

28. Nucleic acid molecule according to any one of claims 24 to 27 characterized in that : it comprises in its sequence a sequence encoding a SU region or a part thereof which comprises at least a part of the amino acid sequence of the DCLV-1-SU9 sequence depicted in figure 9, said part comprising at least one of the substitution or deletion which distinguishes DCLV-1-SU9 from Akv depicted in figure 9.

29. Nucleic acid molecule according to any one of claims 24 to 28 characterized in that : it comprises in its sequence a sequence encoding a SU region or a part thereof which comprises at least a part of the DCLV-1-SU1 amino acid sequence depicted in figure 9, said part comprising at least one of the substitution or deletion which distinguishes DCLV-1-SU1 from the Bxv-1, and/or from NZB-9-1 and/or from MCF247 sequences depicted in figure 9.

30. Nucleic acid molecule according to any one of claims 24 to 29 characterized in that : it comprises a SU region or a part thereof which comprises at least a part of the DCLV-1-SU1 nucleotide sequence depicted in figure 8, said part comprising at least one of the substitution or deletion which distinguishes DCLV-1-SU1 from DBA2 asc and/or from NZB-9-1 and/or from MCF247 and/or from MUSMLPVA sequences depicted in figure 8.

31. Nucleic acid molecule according to any one of claims 24 to 30 characterized in that : it comprises in its sequence all or part of a sequence which can be retrieved from the cDNA of the RNA of a retrovirus of the invention by using a pair of primers chosen among the pair of primers depicted in figure 11.

32. Nucleic acid molecule characterized in that : it is a derivative of a nucleic acid molecule according to any one of claims 24 to 31.

33. Polypeptide characterized in that : it is encoded by a nucleic acid molecule according to any one of claims 22 to 32.

34. Polypeptide characterized in that : it is obtained or isolated from a retrovirus according to any one of claims 1 to 22.

35. Polypeptide according to any one of claims 33 or 34 characterized in that : it comprises a pol region or a part thereof which comprises an amino acid sequence depicted in figure 7 or a part thereof, said part comprising at least the amino acid which differentiates the amino acid sequence of the CMRT-2 of figure 7 from the other amino acid sequences depicted in figure 7.

36. Polypeptide according to any one of claims 33 or 35 characterized in that : it comprises in its sequence a sequence encoding a SU region or a part thereof which comprises at least a part of the DCLV-1-SU1 amino acid sequence depicted in figure 9, said part comprising at least one of the substitution or deletion which distinguishes the DCLV-1-SU1 sequence from the Bxv-1, and/or from NZB-9-1 and/or from MCF247 sequences depicted in figure 9.

37. Vector comprising a nucleic acid molecule according to any one of claims 24 to 32 or a derivative thereof.

38. Vector capable of expressing a polypeptide according to any one of claims 33 to 36 or a part thereof.

39. Cell which comprises a retrovirus according to any one of claims 1 to 22, or a part thereof.

40. Cell which comprises a nucleic acid molecule according to any one of claims 24 to 32 or a derivative thereof, or a part thereof.

41. Cell which comprises a vector according to any one of claims 37 or 38, or a part thereof.

42. DLC 01 cells registered at the CNCM the 17^{th} May 1999 under registry number I-2200.

43. Use of a retrovirus according to any one of claims 1 to 22, or a part thereof as a diagnostic tool.

44. Use of a nucleic acid molecule according to any one of claims 24 to 32 or a derivative thereof as a diagnostic tool.

45. Pharmaceutical composition comprising a retrovirus according to any one of claims 1 to 22, a nucleic acid molecule according to any one of claims 24 to 32, a vector according to any one of claims 37 or 38, and/or a cell according to anyone of claims 39 to 42.

46. Use of a retrovirus according to any one of claims 1 to 22, a nucleic acid molecule according to any one of claims 24 to 32, a vector according to any one of claims 37 or 38, and/or a cell according to any one of claims 39 to 42 to implement a medicament to treat an auto-immune disease , an hematopoietic malignancy or a malignant tumor.


### Annex to the application documents - subsequently filed sequences listing

SEQUENCE LISTING

<110> ETABLISSEMENT DE TRANSFUSION SANGUINE DE LYON

<120> INFECTIOUS RETROVIRUSES FROM A LEUKEMIC DOG CELL LINE
      WITH EXTENSIVE HOMOLOGIES TO MURINE LEUKEMIA VIRUSES

<130> B4279A-GD/LL

<140> 99401199.7
<141> 1999-05-18

<160> 27

<170> PatentIn Ver. 2.1

<210> 1
<211> 75
<212> DNA
<213> Artificial Sequence

<220>
<223> Description of Artificial Sequence:clone
      corresponding to the pol region

<220>
<221> CDS
<222> (1)..(75)

<400> 1
agt ccc acc ctg ttt gat gag gca ctg cac aga gac cta gca ggc ttc    48
Ser Pro Thr Leu Phe Asp Glu Ala Leu His Arg Asp Leu Ala Gly Phe
  1               5                  10                  15

cgg atc cag cac cca gac ttg atc ctg                                75
Arg Ile Gln His Pro Asp Leu Ile Leu
             20                  25

<210> 2
<211> 25
<212> PRT
<213> Artificial Sequence
<223> Description of Artificial Sequence:clone
      corresponding to the pol region

<400> 2
Ser Pro Thr Leu Phe Asp Glu Ala Leu His Arg Asp Leu Ala Gly Phe
  1               5                  10                  15

Arg Ile Gln His Pro Asp Leu Ile Leu
             20                  25

<210> 3
<211> 75
<212> DNA
<213> Artificial Sequence

<220>
<223> Description of Artificial Sequence:clone
      corresponding to the pol region

<220>

<221> CDS
<222> (1)..(75)

<400> 3
agt ccc acc ctg ttt gat gag gca cta cac aga gac cta gca gac ttc    48
Ser Pro Thr Leu Phe Asp Glu Ala Leu His Arg Asp Leu Ala Asp Phe
1               5                   10                  15

cgg atc cag cac cca gac ttg atc ctg                                75
Arg Ile Gln His Pro Asp Leu Ile Leu
            20                  25


<210> 4
<211> 25
<212> PRT
<213> Artificial Sequence
<223> Description of Artificial Sequence:clone
      corresponding to the pol region

<400> 4
Ser Pro Thr Leu Phe Asp Glu Ala Leu His Arg Asp Leu Ala Asp Phe
1               5                   10                  15

Arg Ile Gln His Pro Asp Leu Ile Leu
            20                  25


<210> 5
<211> 75
<212> DNA
<213> Artificial Sequence

<220>
<223> Description of Artificial Sequence:clone
      corresponding to the pol region

<220>
<221> CDS
<222> (1)..(75)

<400> 5
agt ccc acc ctg ttt gat gag gca cta cac aga gac cta gca gac ttc    48
Ser Pro Thr Leu Phe Asp Glu Ala Leu His Arg Asp Leu Ala Asp Phe
1               5                   10                  15

cgg atc cag cac cca gac ttg atc ctg                                75
Arg Ile Gln His Pro Asp Leu Ile Leu
            20                  25


<210> 6
<211> 25
<212> PRT
<213> Artificial Sequence
<223> Description of Artificial Sequence:clone
      corresponding to the pol region

<400> 6
Ser Pro Thr Leu Phe Asp Glu Ala Leu His Arg Asp Leu Ala Asp Phe
1               5                   10                  15

Arg Ile Gln His Pro Asp Leu Ile Leu
            20                  25

<210> 7
<211> 75
<212> DNA
<213> Artificial Sequence

<220>
<223> Description of Artificial Sequence:clone
      corresponding to the pol region

<220>
<221> CDS
<222> (1)..(75)

<400> 7
agt ccc acc ctg ttt gat gag gca ctg cac aga gac cta gca gac ttc    48
Ser Pro Thr Leu Phe Asp Glu Ala Leu His Arg Asp Leu Ala Asp Phe
  1               5                  10                  15

cgg atc cag cac cca gac ttg atc ctg                                75
Arg Ile Gln His Pro Asp Leu Ile Leu
             20                  25

<210> 8
<211> 25
<212> PRT
<213> Artificial Sequence
<223> Description of Artificial Sequence:clone
      corresponding to the pol region

<400> 8
Ser Pro Thr Leu Phe Asp Glu Ala Leu His Arg Asp Leu Ala Asp Phe
  1               5                  10                  15

Arg Ile Gln His Pro Asp Leu Ile Leu
             20                  25

<210> 9
<211> 491
<212> DNA
<213> Artificial Sequence

<220>
<223> Description of Artificial Sequence:clone SU1
      corresponding to the env region

<400> 9
cacgccgctc acgtaaaagc ggcgacaacc cctccggccg gaacagcatc aggaccgaca 60

tggaaggtcc agcgttctca aaatccctta aagataagat taacccgtgg ggccccctga 120

tagttatggg gaccttagtg agggcaggag cctcggtaca acgtgacagc cctcaccagg 180

tcttcaatgt tacttggaga gttaccaacc taatgacagg acaaacagct aacgccacct 240

ccctcctggg gacgatgaca gacaccttcc ctaaactata ttttgacctg tgtgatttag 300

taggagacta ctgggatgac ccagatggag atggttgccg ctctcccggg ggaagaaaaa 360

ggacaagact gtatgacttc tatgtttgcc ccggtcatac tgtaccaata gggtgtggag 420

ggccgggaga gggctactgt ggcaaatggg gatgtgagac cactggacag gcatactgga 480

agccatcatc a 491

<210> 10
<211> 337
<212> PRT
<213> Artificial Sequence

<220>
<223> Description of Artificial Sequence:protein
sequence of clone SU1 corresponding to the env
region

<400> 10
Met Glu Gly Pro Ala Phe Ser Lys Ser Leu Lys Asp Lys Ile Asn Pro
1               5                   10                  15

Trp Gly Pro Leu Ile Val Met Gly Thr Leu Val Arg Ala Gly Ala Ser
            20                  25                  30

Val Gln Arg Asp Ser Pro His Gln Val Phe Asn Val Thr Trp Arg Val
        35                  40                  45

Thr Asn Leu Met Thr Gly Gln Thr Ala Asn Ala Thr Ser Leu Leu Gly
    50                  55                  60

Thr Met Thr Asp Thr Phe Pro Lys Leu Tyr Phe Asp Leu Cys Asp Leu
65                  70                  75                  80

Val Gly Asp Tyr Trp Asp Asp Pro Asp Gly Asp Gly Cys Arg Ser Pro
                85                  90                  95

Gly Gly Arg Lys Arg Thr Arg Leu Tyr Asp Phe Tyr Val Cys Pro Gly
            100                 105                 110

His Thr Val Pro Ile Gly Cys Gly Gly Pro Gly Glu Gly Tyr Cys Gly
        115                 120                 125

Lys Trp Gly Cys Glu Thr Thr Gly Gln Ala Tyr Trp Lys Pro Ser Ser
    130                 135                 140

Ser Trp Asp Leu Ile Ser Leu Lys Arg Gly Asn Thr Ser Lys Asp Xaa
145                 150                 155                 160

Gly Pro Xaa Tyr Asp Ser Ser Val Xaa Ser Gly Val Gln Gly Ala Thr
                165                 170                 175

Pro Gly Gly Arg Cys Asn Pro Leu Val Leu Glu Phe Thr Asp Ala Gly
            180                 185                 190

Lys Lys Ala Ser Trp Asp Ala Pro Lys Val Trp Gly Leu Arg Leu Tyr
        195                 200                 205

Arg Ser Thr Gly Ala Asp Pro Val Thr Arg Phe Ser Leu Thr Arg Arg
    210                 215                 220

Val Leu Asn Val Gly Pro Arg Val Pro Ile Gly Pro Asn Pro Val Ile
225                 230                 235                 240

Thr Asp Gln Leu Pro Pro Ser Gln Pro Val Gln Ile Met Leu Pro Arg
                245                 250                 255

Pro Pro His Pro Pro Pro Ser Gly Thr Val Ser Met Val Pro Gly Ala
            260                 265                 270

Pro Pro Pro Ser Gln Gln Pro Gly Thr Gly Asp Arg Leu Leu Asn Leu
        275                 280                 285

Val Asp Gly Ala Tyr Gln Ala Leu Asn Leu Thr Ser Pro Asp Arg Thr
    290                 295                 300

Gln Glu Cys Trp Leu Cys Leu Val Ser Gly Pro Pro Tyr Tyr Glu Gly
305                 310                 315                 320

Val Ala Val Leu Gly Thr Tyr Ser Asn His Thr Ser Ala Pro Ala Asn
                325                 330                 335

Cys

<210> 11
<211> 385
<212> PRT
<213> Artificial Sequence

<220>
<223> Description of Artificial Sequence:protein
      sequence of clone SU9 corresponding to the env
      region

<400> 11
Met Asp Xaa Arg Arg Pro Arg Gln Gly Ser Asp His Ala Pro Asp Lys
  1               5                  10                  15

Thr Ile Met Glu Ser Thr Thr Leu Ser Lys Pro Phe Lys Asn Gln Val
             20                  25                  30

Asn Pro Trp Gly Pro Leu Ile Val Leu Leu Ile Leu Gly Gly Val Asn
         35                  40                  45

Pro Val Thr Leu Gly Asn Ser Pro His Gln Val Phe Asn Leu Thr Trp
     50                  55                  60

Glu Val Thr Asn Gly Asp Arg Glu Thr Val Trp Ala Ile Thr Gly Asn
 65                  70                  75                  80

His Pro Leu Trp Thr Trp Trp Pro Asp Leu Thr Pro Asp Leu Cys Met
                 85                  90                  95

Leu Ala Leu His Gly Pro Ser Tyr Trp Gly Leu Glu Tyr Arg Ala Pro
            100                 105                 110

Phe Ser Pro Pro Pro Gly Pro Pro Cys Cys Ser Gly Ser Ser Asp Ser
        115                 120                 125

Thr Pro Gly Cys Ser Arg Asp Cys Glu Glu Pro Leu Thr Ser Tyr Thr
    130                 135                 140

Pro Arg Cys Asn Thr Ala Trp Asn Arg Leu Lys Leu Ser Lys Val Thr
145                 150                 155                 160

His Ala His Asn Gly Gly Phe Tyr Val Cys Pro Gly Pro His Arg Pro
                165                 170                 175

Arg Trp Ala Arg Leu Cys Gly Gly Pro Lys Ser Phe Tyr Cys Ala Ser

180                 185                 190

Trp Gly Cys Glu Thr Ala Gly Arg Ala Ser Trp Lys Pro Ser Ser Ser
        195                 200                 205

Trp Asp Tyr Ile Thr Val Ser Asn Asn Leu Thr Ser Asp Gln Ala Thr
    210                 215                 220

Pro Val Cys Lys Gly Asn Glu Trp Cys Asn Ser Leu Ile Ile Arg Phe
225                 230                 235                 240

Thr Ser Phe Gly Lys Gln Ala Thr Ser Trp Val Thr Gly His Trp Trp
                245                 250                 255

Gly Leu Arg Leu Tyr Val Ser Gly His Asp Pro Gly Leu Ile Phe Gly
            260                 265                 270

Ile Arg Leu Lys Ile Thr Asp Ser Gly Pro Arg Val Pro Ile Gly Pro
        275                 280                 285

Asn Pro Val Leu Ser Asp Arg Arg Pro Pro Ser Arg Pro Arg Pro Thr
    290                 295                 300

Arg Ser Pro Pro Pro Ser Asn Ser Thr Pro Thr Glu Thr Pro Leu Thr
305                 310                 315                 320

Leu Pro Glu Pro Pro Pro Ala Gly Val Glu Asn Arg Leu Leu Asn Leu
                325                 330                 335

Val Lys Gly Ala Tyr Gln Ala Leu Asn Leu Thr Ser Pro Asp Lys Thr
            340                 345                 350

Gln Glu Cys Trp Leu Cys Leu Val Ser Gly Pro Pro Tyr Tyr Glu Gly
        355                 360                 365

Val Ala Val Leu Gly Thr Tyr Ser Asn His Thr Ser Ala Pro Ala Asn
    370                 375                 380

Cys
385

<210> 12
<211> 21
<212> DNA
<213> Artificial Sequence

<220>
<223> Description of Artificial Sequence:primer

<400> 12
ctyccncagg gkttcaaraa c                                            21

<210> 13
<211> 18
<212> DNA
<213> Artificial Sequence

<220>
<223> Description of Artificial Sequence:primer

<400> 13
gtcatcyacr taytgkag                                                18

<210> 14
<211> 20
<212> DNA
<213> Artificial Sequence

<220>
<223> Description of Artificial Sequence:primer

<400> 14
ctgaacccag gataaccctc                                                    20


<210> 15
<211> 20
<212> DNA
<213> Artificial Sequence

<220>
<223> Description of Artificial Sequence:primer

<400> 15
ggatcaagtc tgggtgctgg                                                    20


<210> 16
<211> 20
<212> DNA
<213> Artificial Sequence

<220>
<223> Description of Artificial Sequence:primer

<400> 16
cccaccctgt ttgatgaggc                                                    20


<210> 17
<211> 20
<212> DNA
<213> Artificial Sequence

<220>
<223> Description of Artificial Sequence:primer

<400> 17
gcccagatta cctcggtctc                                                    20


<210> 18
<211> 20
<212> DNA
<213> Artificial Sequence

<220>
<223> Description of Artificial Sequence:primer

<400> 18
tgaccacccc caccgctctc                                                    20


<210> 19
<211> 19

<212> DNA
<213> Artificial Sequence

<220>
<223> Description of Artificial Sequence:primer

<400> 19
gagcagttag ctggggcag                                                    19


<210> 20
<211> 21
<212> DNA
<213> Artificial Sequence

<220>
<223> Description of Artificial Sequence:primer

<400> 20
aagagtgctg gttatgccta g                                                 21


<210> 21
<211> 19
<212> DNA
<213> Artificial Sequence

<220>
<223> Description of Artificial Sequence:primer

<400> 21
cagtctatcg gaggactgg                                                    19


<210> 22
<211> 26
<212> DNA
<213> Artificial Sequence

<220>
<223> Description of Artificial Sequence:primer

<400> 22
tgrcartcrt cccargtngg ytgrtg                                            26


<210> 23
<211> 18
<212> DNA
<213> Artificial Sequence

<220>
<223> Description of Artificial Sequence:primer

<400> 23
tgggggctcg tccgggat                                                     18


<210> 24
<211> 18
<212> DNA
<213> Artificial Sequence

<220>

<223> Description of Artificial Sequence:primer

<400> 24
atcccggacg agccccca                                                    18

<210> 25
<211> 19
<212> DNA
<213> Artificial Sequence

<220>
<223> Description of Artificial Sequence:primer

<400> 25
accgattact gtgtcctgg                                                   19

<210> 26
<211> 19
<212> DNA
<213> Artificial Sequence

<220>
<223> Description of Artificial Sequence:primer

<400> 26
cagcatcagg accgacatg                                                   19

<210> 27
<211> 20
<212> DNA
<213> Artificial Sequence

<220>
<223> Description of Artificial Sequence:primer

<400> 27
gwgcggcaac catctccatc                                                  20